# EUROPÄISCHE PATENTSCHRIFT

(11) **EP 1 102 768 B1**
(45) Veröffentlichungstag und Bekanntmachung des Hinweises auf die Patenterteilung: **21.12.2005**
(21) Anmeldenummer: 99938273.2
(22) Anmeldetag: 16.07.1999
(51) Int. Cl.: C07D 471/04, A61K 31/435

(54) **MIT SECHSGLIEDRIGEN HETEROCYCLISCHEN RINGEN KONDENSIERTE SUBSTITUIERTE PYRAZOLDERIVATE**
SUBSTITUTED PYRAZOLE DERIVATIVES CONDENSED WITH SIX-MEMBERED HETEROCYCLIC RINGS
DERIVES DE PYRAZOLE SUBSTITUES, CONDENSES AVEC DES NOYAUX HETEROCYCLIQUES A SIX CHAINES

(30) Priorität: 29.07.1998 DE 19834044
(43) Veröffentlichungstag der Anmeldung: 30.05.2001
(73) Patentinhaber: Bayer HealthCare AG, 51368 Leverkusen (DE)
(72) Erfinder: STRAUB, Alexander, D-42113 Wuppertal (DE); FEURER, Achim, D-51519 Odenthal (DE); ALONSO-ALIJA, Cristina, D-42781 Haan (DE); STASCH, Johannes-Peter, D-42651 Solingen (DE); PERZBORN, Elisabeth, D-42327 Wuppertal (DE); HÜTTER, Joachim, D-42349 Wuppertal (DE); DEMBOWSKY, Klaus, D-69198 Schriesheim (DE); STAHL, Elke, D-51467 Bergisch Gladbach (DE)
(86) Internationale Anmeldenummer: PCT/EP1999/005074
(87) Internationale Veröffentlichungsnummer: WO 2000/006569

(56) Entgegenhaltungen:
- EP-A- 0 086 422
- EP-A- 0 667 345
- WO-A-98/16507
- WO-A-98/23619

## Beschreibung

Die vorliegende Erfindung betrifft neue substituierte Pyrazol-Derivate, Verfahren zu ihrer Herstellung und ihre Verwendung als Arzneimittel, insbesondere als Arzneimittel zur Behandlung von Herz-Kreislauf-Erkrankungen nach den Ansprüchen 1 bis 10.

Es ist bereits bekannt, daß 1-Benzyl-3-(substituierte heteroaryl)-kondensierte Pyrazol-Derivate die Thrombozytenaggregation inhibieren (vgl. EP 667 345 Al).

WO 98/16223 offenbart die Verwendung von 1-Benzyl-3-(substituiertes-Hetaryl)-kondensierten Pyrazolderivaten zur Behandlung von speziellen Erkrankungen des Herz-Kreislaufsystems und des Zentralnervensystems.

WO 98/16507 offenbart Heterocyclylmethyl-substituierte Pyrazolderivate und ihre Verwendung bei der Behandlung von Herz-Kreislauf-Erkrankungen.

WO 98/23619 offenbart ebenfalls substituierte Pyrazolderivate zur Behandlung von Herz-Kreislauf-Erkrankungen.

EP-A-0 086 422 offenbart Pyrazolo (4,3- C) pyridin- Derivate mit breiter pharmakologischer Wirkung.

Die vorliegende Erfindung betrifft substituierte Pyrazolderivate der allgemeinen Formel (I) 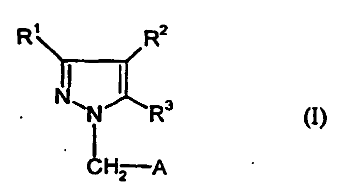 in welcher
- R² und R³: unter Einbezug der Doppelbindung einen Pyridyl-oder Pyrimidinylring bilden,
- A: für Phenyl oder Pyrimidyl steht, die gegebenenfalls durch Fluor, Chlor oder Brom substituiert sind,
- R¹: für einen Rest der Formel 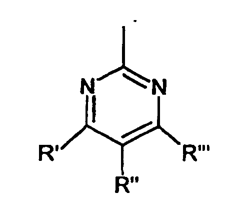
steht, worin
- R': für NH₂ steht
- R": für gegebenenfalls substituiertes Morpholino, Piperidin, Piperazin, Pyrrolidin, Triazolyl oder Thiomorpholino steht
und
- R''': für Wasserstoff oder NH₂ steht.

Bevorzugt sind hierbei Verbindungen, bei denen R" für Morpholinyl steht.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweiligen Mischungen. Die Racemformen lassen sich ebenso wie die Diastereomeren in bekannter Weise in die stereoisomer einheitlichen Bestandteile trennen.

Im Rahmen der vorliegenden Erfindung haben die Substituenten soweit nicht anders angegeben im allgemeinen die folgende Bedeutung:

Weiterhin ist Gegenstand der Erfindung ein Verfahren zur Herstellung von Verbindungen der allgemeinen Formel (I), bei dem man in Abhängigkeit der verschiedenen Bedeutungen der oben unter R² und R³ aufgeführten Heterocyclen
[A] Verbindungen der allgemeinen Formel (II) (II)

   R¹-D (II)

   in welcher
   - R¹: die oben angegebene Bedeutung hat,
   und
   - D: für Reste der Formeln 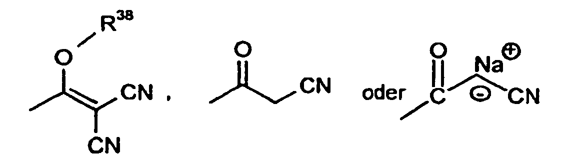
   steht,
   in welchen
   - R³⁸: für C₁-C₄-Alkyl steht,
   durch Umsetzung mit Verbindungen der allgemeinen Formel (III)

   A-CH₂-NH-NH₂ (III)

   in welcher
   - A: die oben angegebene Bedeutung hat
   in inerten Lösemitteln, gegebenenfalls in Anwesenheit einer Base, in die Verbindungen der allgemeinen Formel (IV) oder (IVa) 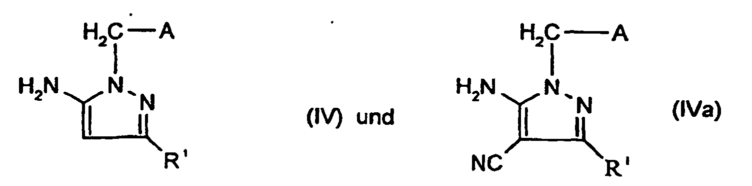 in welcher
   - A und R¹: die oben angegebene Bedeutung haben,
   überführt,
   und im Fall der Verbindungen der allgemeinen Formel (IVa) anschließend mit Carbonsäuren, Nitrilen, Formamiden oder Guanidiniumsalzen cyclisiert,
   und im Fall der Verbindungen der allgemeinen Formel (IV) mit 1,3-Dicarbonyl-Derivaten, deren Salze, Tautomeren, Enolether oder Enaminen, in Anwesenheit von Säuren und gegebenenfalls unter Mikrowellen cyclisiert,
   oder
[C] Verbindungen der allgemeinen Formel (VII) 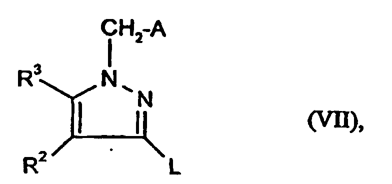 in welcher
   - A', R² und R³: die oben angegebene Bedeutung haben,
   und
   - L: für einen Rest der Formel -SnR³⁹R⁴⁰R⁴¹, ZnR⁴², Iod, Brom oder Triflat steht,
   worin
   - R³⁹, R⁴⁰ und R⁴¹: gleich oder verschieden sind und geradkettiges oder verzweigtes Alkyl mit bis zu 4 Kohlenstoffatomen bedeuten,
   und
   - R⁴²: Halogen bedeutet,
   mit Verbindungen der allgemeinen Formel (VIII) (VIII),

   R¹-T (VIII)

   in welcher
   - R¹: die oben angegebene Bedeutung hat
   und
   im Fall L = SnR³⁹R⁴⁰R⁴¹ oder ZnR⁴²
   - T: für Triflat oder für Halogen, vorzugsweise für Brom steht,
   und
   im Fall L = Iod, Brom oder Triflat
   - T: für einen Rest der Formel SnR³⁹R⁴⁰R⁴¹', ZnR⁴²' oder BR⁴³'R⁴⁴' steht,
   worin
   R^{39'}, R^{40'}, R^{41'} und R^{42'} die oben angebene Bedeutung von R³⁹, R⁴⁰, R⁴¹ und R⁴² haben und mit diesen gleich oder verschieden sind,
   - R^{43'} und R^{44'}: gleich oder verschieden sind und Hydroxy, Aryloxy mit 6 bis 10 Kohlenstoffatomen oder geradkettiges oder verzweigtes Alkyl oder Alkoxy mit jeweils bis zu 5 Kohlenstoffatomen bedeuten, oder gemeinsam einen 5- oder 6-gliedrigen carbocyclischen Ring bilden,
   in einer palladiumkatalysierten Reaktion in inerten Lösemitteln umsetzt, gegebenenfalls in Gegenwart einer Base, [D] im Fall, daß R¹ für einen gegebenenfalls substituierten Pyrimidinrest steht, Amidine der allgemeinen Formel (IX) 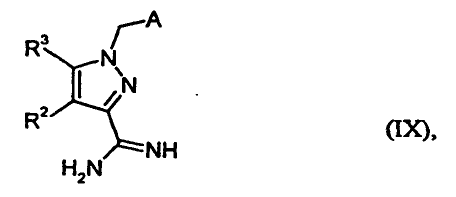 in welcher
   - A, R² und R³: die oben angegebene Bedeutung haben,
   beispielsweise mit Verbindungen der allgemeinen Formel (X), (Xa), (Xb) oder (Xc) 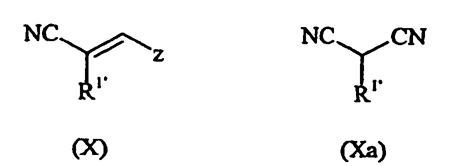 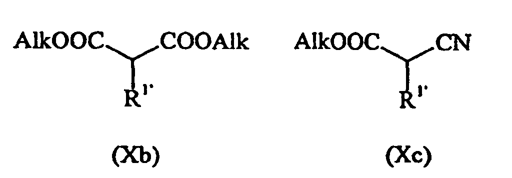 in welchen
   - R¹: für den oben unter R¹ aufgeführten gegebenenfalls substituierten Cycloalkylrest steht;
   - Alk: für geradkettiges oder verzweigtes Alkyl mit bis zu 8 Kohlenstoffatomen, vorzugsweise bis zu vier Kohlenstoffatomen steht;
   oder
   und
   - Z: für eine NH₂-Gruppe, Monoalkylaminogruppe mit bis zu 7 Kohlenstoffatomen, Dialkylaminogruppe mit bis zu 7 Kohlenstoffatomen, einen über den Stickstoff gebundenen Piperidino- oder Morpholinorest, Hydroxyl, Alkoxy mit bis zu 7 Kohlenstoffatomen, Acyloxy mit bis zu 7 Kohlenstoffatomen oder Aroyloxy mit 6 bis 10 Kohlenstoffatomen steht,
   umsetzt,
   und gegebenenfalls die unter X, Y, R¹, R², R³ und/oder A aufgeführten Substituenten nach üblichen Methoden, vorzugsweise durch Acylierung freier Aminogruppen oder Hydroxygruppen, Chlorierung, katalytische Hydrierung, Reduktion, Oxidation, Abspaltung von Schutzgruppen und/oder nucleophiler Substitution variiert oder einfühzt.

Das erfindungsgemäße Verfahren kann durch folgende Formelschemata beispielhaft erläutert werden:

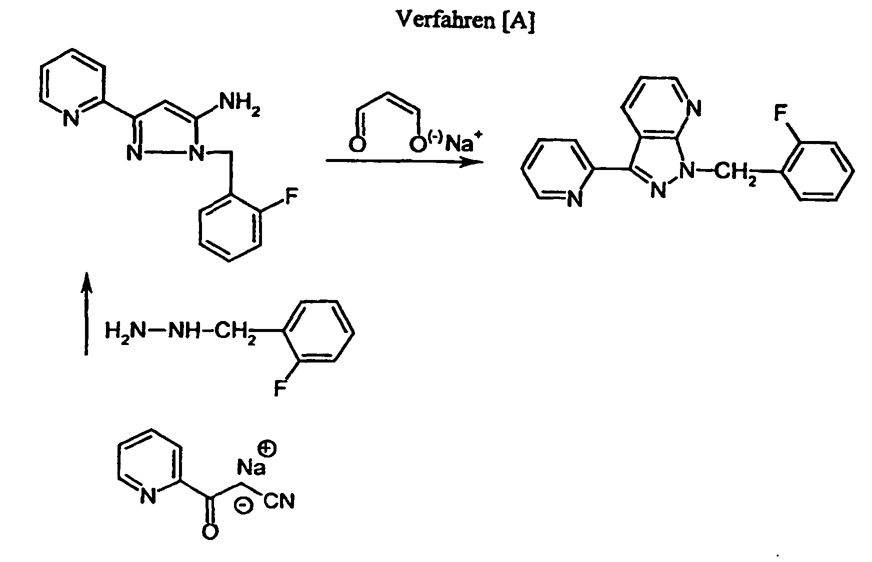

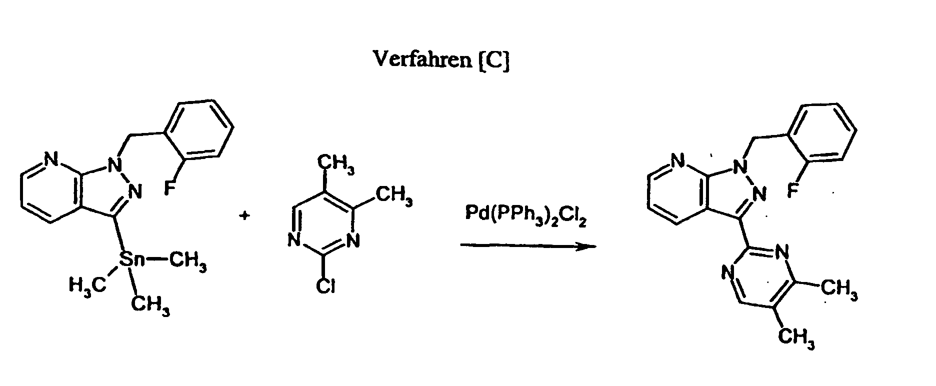

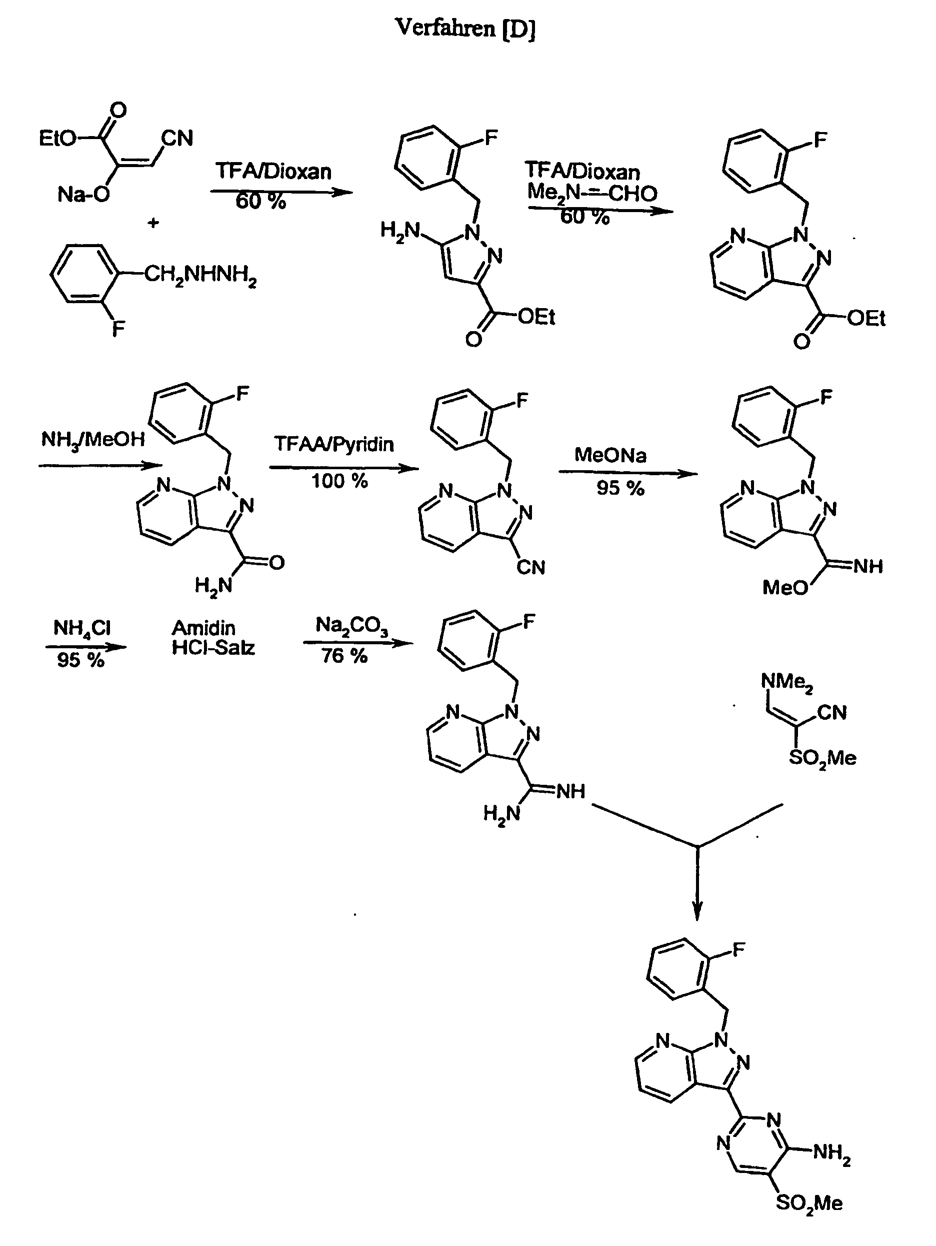

Die unter R² und R³ aufgeführten Heterocyclen können auch durch Umsetzung der entsprechend substituierten Verbindungen nach anderen bekannten heterocyclischen Synthesen eingeführt werden.

Als Lösemittel für die einzelnen Schritte des Verfahrens [A] eignen sich hierbei inerte organische Lösemittel, die sich unter den Reaktionsbedingungen nicht verändern. Hierzu gehören Ether, wie Diethylether oder Tetrahydrofuran, DME, Dioxan, Alkohole wie Methanol und Ethanol, Halogenköhlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt ist Tetrahydrofuran, Dimethylformamid, Toluol, Dioxan oder Dimethoxyethan.

Als Basen können bei den erfindungsgemäßen Verfahren im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)-amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat, Triethylamin und Natriumhydrid.

Die Base wird bei der Umsetzung der Verbindungen der Formel (II) mit den Verbindungen der Formel (III) in einer Menge von 1 mol bis 5 mol, bevorzugt von 1 mol bis 3 mol, bezogen auf 1 mol der Verbindung der allgemeinen Formel (II) eingesetzt.

Die Umsetzung der Verbindungen der Formel (III) mit den Verbindungen der Formel (III) wird im allgemeinen in einem Temperaturbereich von 0°C bis 150°C, bevorzugt von +20°C bis +110°C durchgeführt.

Diese Umsetzung kann bei normalen, erhöhtem oder bei erniedrigtem Druck durchgeführt werden (zB. 0,5 bis 5 bar). Im allgemeinen arbeitet man bei Normaldruck.

Als Säuren für die gegebenenfalls bei den erfindungsgemäßen Verfahren durchzuführenden Cyclisierungsreaktionen eignen sich im allgemeinen Protonensäuren. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder Arylresten wie beispielsweise Methansulfonsäure, Ethansulfonsäure, Benzolsulfonsäure oder Toluolsulfonsäure.

Die gegebenenfalls innerhalb der erfindungsgemäßen Verfahren durchzuführenden katalytischen Hydrierungsreaktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Die gegebenenfalls innerhalb der erfindungsgemäßen Verfahren durchzuführenden Chlorierungsreaktionen erfolgen im allgemeinen mit den üblichen Chlorierungsmitteln wie beispielsweise PCl₃, PCl₅, POCl₃ oder elementarem Chlor. Bevorzugt ist im Rahmen der Erfindung POCl₃.

Die gegebenenfalls innerhalb der erfindungsgemäßen Verfahren durchzuführenden Acylierungen freier Aminogruppen oder Hydroxygruppen können nach üblichen, dem Fachmann geläufigen Methoden durchgeführt werden. Beispielsweise können entsprechende freie Aminogruppen oder Hydroxygruppen durch Umsetzung mit einem Säurehalogenid, vorzugsweise einem Säurechlorid, oder mit einem Säureanhydrid in Gegenwart einer Base wie beispielsweise Natriumhydrid, Pyridin oder Dimethylaminopyridin in einem Lösungsmittel wie Tetrahydrofuran oder Dichlormethan in die jeweiligen Amide oder Ester oder durch Umsetzung mit einem Sulfonylhalogenid, vorzugsweise einem Sulfonylchlorid, in die jeweiligen Sulfonamide oder Sulfonsäureester überführt werden.

Die gegebenenfalls innerhalb der erfmdungsgemäßen Verfahren durchzuführenden Oxidationen von Thioethergruppen zu Sulfoxidgruppen beziehungsweise Sulfongruppen können nach üblichen, dem Fachmann geläufigen Methoden durchgeführt werden. Beispielsweise können derartige Oxidationen mit m-Chlorperoxybenzoesäure (MCPBA) in einem Lösungsmittel wie Dichlormethan durchgeführt werden.

Die gegebenenfalls innerhalb der erfindungsgemäßen Verfahren durchzuführenden nucleophilen Substitutionen und Vilsmeierreaktionen werden nach üblichen, dem Fachmann geläufigen Methoden durchgeführt.

Die gegebenenfalls innerhalb der erfindungsgemäßen Verfahren durchzuführenden Reduktionen werden im allgemeinen mit Reduktionsmitteln, bevorzugt mit solchen, die für die Reduktion von Carbonyl zu Hydroxyverbindungen geeignet sind, durchgeführt. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithiumtrialkylhydrido-boranat, Diisobutylaluminiumhydrid oder Lithiumaluminiumhydrid durchgeführt. Ganz besonders bevorzugt wird die Reduktion mit Diisobutylalumimumhydrid und Natriumborhydrid durchgeführt.

Das Reduktionsmittel wird hierbei im allgemeinen in einer Menge von 1 mol bis 6 mol, bevorzugt von 1 mol bis 4 mol bezogen auf 1 mol der zu reduzierenden Verbindungen, eingesetzt.

Die gegebenenfalls innerhalb der erfindungsgemäßen Verfahren durchzuführenden Reduktionen werden im allgemeinen in einem Temperaturbereich von -78°C bis +50°C durchgeführt, bevorzugt von -78°C bis 0°C im Falle des DIBAH und 0°C bis Raumtemperatur im Falle des NaBH₄.

Die gegebenenfalls innerhalb der erfindungsgemäßen Verfahren durchzuführenden Reduktionen werden im allgemeinen bei Normaldruck durchgerührt. Es ist aber auch möglich, bei erhöhtem oder erniedrigtem Druck zu arbeiten.

Die Verbindungen der allgemeinen Formeln (II) und (III) sind an sich bekannt oder nach üblichen Methoden herstellbar (vgl. hierzu: J. Hromatha et al., Monatsh. Chem. 1976, 107, 233).

Die Verbindungen der allgemeinen Formeln (IV), und (IVa) sind teilweise bekannt und können wie oben beschrieben hergestellt werden.

Als Lösemittel für das Verfahren [C] eignen sich inerte organische Lösemittel, die sich unter den Reaktionsbedingungen nicht verändern. Hierzu gehören Ether, wie Diethylether oder Tetrahydrofuran, DME, Dioxan, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt ist Tetrahydrofuran, Dimethylformamid, Toluol, Dioxan oder Dimethoxyethan.

Die Umsetzung der Verbindungen der Formel (VII) mit den Verbindungen der Formel (VIII) wird im allgemeinen in einem Temperaturbereich von 0°C bis 150°C, bevorzugt von +20°C bis +110°C durchgeführt.

Diese Umsetzung kann bei normalen, erhöhtem oder bei erniedrigtem Druck durchgeführt werden (z.B. 0,5 bis 5 bar). Im allgemeinen arbeitet man bei Normaldruck.

Als Palladiumverbindungen im Rahmen der vorliegenden Erfindung eignen sich im allgemeinen PdCl₂(P(C₆H₅)₃)₂, Palladium-bis-dibenzylidenaceton (Pd(dba)₂), [1,1'-Bis-(diphenylphosphino)ferrocen]-Palladium(II)-chlorid (Pd(dppf)Cl₂) oder Pd(P(C₆H₅)₃)₄. Bevorzugt ist Pd(P(C₆H₅)₃)₄.

Die Verbindungen der allgemeinen Formel (VII) sind an sich bekannt oder nach üblichen Methoden herstellbar (vgl. beispielsweise K. Kirschke in: Houben-Weyl, Methoden der organischen Chemie, Thieme-Verlag Stuttgart, 4. Aufl., Band E8b, Teil 2, 399-763; insbesondere bezüglich Pyrazolopyridinen: C.R. Hardy in A.R. Katritzky (Hrsg.), Adv. Het. Chem. 1984, 36, 343-409; insbesondere bezüglich Pyrazolopyrimidinen: M.H. Elgnadi et al., Adv. Het. Chem. 1987, 41, 319-376). Die Herstellung der entsprechenden Halogenpyrazolo[3,4-b]pyrimidine und zinnorganischen Pyrazolo[3,4-b]pyrimidine der Formel (VII) ist in der WO 98/23619 beschrieben und kann analog auch für die entsprechenden Triflat- und zinkorganischen Verbindungen der Formel (VII) durchgeführt werden.

Die Verbindungen der allgemeinen Formel (VIII) sind bekannt und nach üblichen Methoden herstellbar (vgl. beispielsweise M.G. Hoffmann et al. in: Houben-Weyl, Methoden der organischen Chemie, 4. Aufl., Band E9b, Teil 1, S. 1-249; A.

Weissenberger et al., The Chemistry of heterocyclic compounds - Pyrimidines, 1962, 16; ibid 1970, 16, Suppl. 1, ibid 1985, 16, Suppl. 2; ibid 1994, 52).

Das Verfahren [D] wird in einem Temperaturbereich von 80°C bis 120°C, vorzugsweise bei 100°C bis 110°C oder unter Rückfluß durchgeführt.

Als Lösemittel können beispielsweise die Reagentien der allgemeinen Formel (X), (Xa), (Xb) oder (Xc) fungieren. Die Reaktion kann aber auch in anderen geeigneten Lösemitteln durchgeführt werden, wie z.B. Toluol, Methanol oder Dichlormethan. Leichtsiedende Lösungsmittel wie z.B. Dichlormethan können im Laufe der Reaktion abdestilliert werden.

Das Verfahren [D] kann bei normalen, erhöhtem oder bei erniedrigtem Druck durchgeführt werden (z.B. 0,5 bis 5 bar). Im allgemeinen arbeitet man bei Normaldruck.

Die Reaktion kann hierbei entweder in einem Schritt oder über offenkettige Verbindungen wie beispielsweise 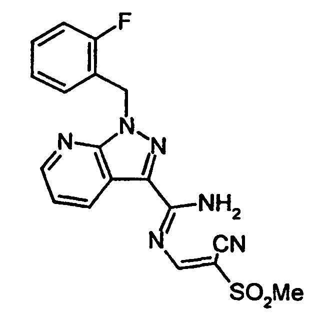 ablaufen. Sie kann im Vakuum durchgeführt werden. Sie kann sowohl mit wie ohne Zusatz der vorstehend aufgeführten Lösungsmittel, Base oder Säure ablaufen.

Die Amidine der allgemeinen Formel (IX) können hergestellt werden, indem man die Verbindungen der allgemeinen Formel (XI) 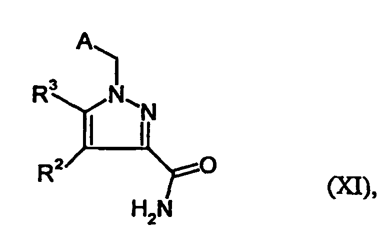 in welcher
- A, R² und R³: die oben angegebene Bedeutung haben,
zunächst in Ethern mit Trifluoressigsäureanhydrid (TFAA) und in Anwesenheit von Basen zu der Verbindung der allgemeinen Formel (XII) 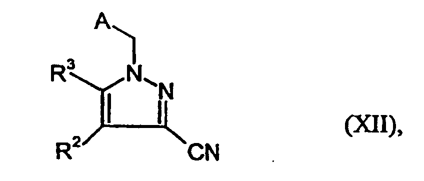 in welcher
- A, R² und R³: die oben angegebene Bedeutung haben,
umsetzt,
anschließend mit Natriummethanolat die Verbindungen der allgemeinen Formel (XIII) 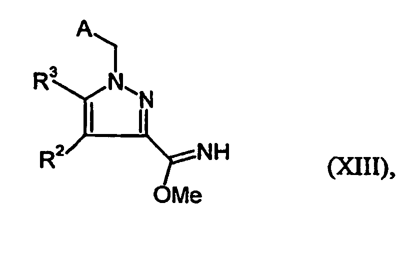 in welcher
- A, R² und R³: die oben angegebene Bedeutung haben,
herstellt, in einem nächsten Schritt durch Umsetzung mit NH₄Cl und Eisessig in Alkoholen in das entsprechende Amidin HCl-Salz der allgemeinen Formel (XIV) 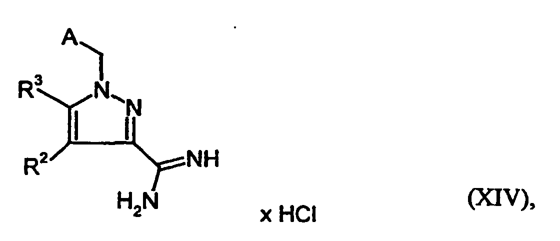 in welcher
- A, R² und R³: die oben angegebene Bedeutung haben,
überführt und in einem letzten Schritt mit Basen, vorzugsweise Natriumcarbonat oder Alkalialkoholat wie Natriumethanolat umsetzt.

Als Lösemittel für Umsetzung der Verbindungen der allgemeinen Formel (XI) zu den Verbindungen der Formel (XII) eignen sich Ether, wie Diethylether oder Tetrahydrofuran, Dimethylformamid und Dioxan; bevorzugt ist Tetrahydrofuran.

Als Basen können hierbei organische Amine (Trialkyl(C₁-C₆)-amine) wie Triethylamin, oder Heterocyclen wie 1,4-Diazabicyclo[2.2.2]octan (DABCO), 1,8-Diazabicyclo[5.4.0]undec-7-en (DBU), Pyridin, Dimethylaminopyridin, Methylpiperidin oder Morpholin eingesetzt werden. Bevorzugt ist Pyridin.

Die Umsetzung erfolgt in einem Temperaturbereich von 0°C bis 40°C, vorzugsweise bei Raumtemperatur.

Die Umsetzung kann bei normalen, erhöhtem oder bei erniedrigtem Druck durchgeführt werden (z.B. 0,5 bis 5 bar). Im allgemeinen arbeitet man bei Normaldruck.

Das Amid (XI) kann beispielsweise durch Verseifung eines entsprechenden Esters als Ausgangsverbindung mit einer Base zur Säure, deren Überführung in das Säurechlorid nach üblichen Methoden z.B. mittels SOCl₂ oder POCl₃ und anschließender Umsetzung mit Ammoniak erfolgen.

Die Eliminierung von Wasser aus dem Amid (XI) zum Nitril (XII) kann mit allen üblichen wasserentziehenden Mitteln durchgeführt werden. Erfindungsgemäß bevorzugt ist Trifluoressigsäureanhydrid (TFAA).

Die Überführung des Nitrils der Formel (XII) in den Iminoether der Formel (XIII) kann sowohl im Sauren, wie z.B. mit HCl/Alkohol-Gemischen, als auch im Basischen wie z.B. mit Methanol/Natriummethanolat erfolgen. Sie erfolgt üblicherweise bei 0°C bis 40°C, beispielsweise bei Raumtemperatur.

Als Lösemittel für Umsetzung der Verbindungen der allgemeinen Formel (XIII) zu den Verbindungen der Formel (XIV) eignen sich Alkohole wie Methanol oder Ethanol. Bevorzugt ist Methanol.

Die Umsetzung erfolgt in einem Temperaturbereich von 0°C bis 40°C, vorzugsweise bei Raumtemperatur.

Die Umsetzung kann bei normalem, erhöhtem oder bei erniedrigtem Druck durchgeführt werden (z.B. 0,5 bis 5 bar). Im allgemeinen arbeitet man bei Normaldruck.

Als Basen für die Freisetzung der Verbindungen der allgemeinen Formel (IX) aus Verbindungen der allgemeinen Formel (XIV) eignen sich anorganische oder organische Basen. Hierzu gehören beispielsweise Alkalihydroxide wie Natriumhydroxid oder Kaliumhydroxid, Erdalkalihydroxide wie Bariumhydroxid, Alkalicarbonate wie Natriumcarbonat oder Kaliumcarbonat, Erdalkalicarbonate wie Calciumcarbonat und Alkalialkoholate wie Natriummethanolat. Bevorzugt sind Natriumcarbonat und Natriummethanolat.

Die Darstellung des Pyrimidinrings erfolgt nach den üblichen Methoden (vgl. beispielsweise M.G. Hoffmann et al. in: Houben-Weyl, Methoden der organischen Chemie, 4.Aufl., Band E9b, Teil 1, S. 1-249; A. Weissenberger et al., The Chemistry of heterocyclic compounds - Pyrimidines, 1962, 16; ibid 1970, 16, Suppl. 1, ibid 1985, 16, Suppl. 2; ibid 1994, 52).

Hierbei kann man von den Iminoethern der Formel (XIII) ausgehen und diesen z.B. mit einem geeigneten Enamin umsetzen. Man kann aber auch den Iminoether zunächst mittels Ammoniak oder dessen Salzen in ein entsprechendes Amidin überführen und dieses entweder als freie Base (IX) oder als Salz (XIV) mit Enaminen, Acetalen, Enolethern, Aldehyden, Enolaten, Malonitrilestern oder Malondinitrilen umsetzen.

Die bei dieser Umsetzung gegebenenfalls einzusetzenden Enamine können z.B. aus C-H-aciden Verbindungen wie Acetonitrilderivaten nach bekannten Methoden durch Umsetzung mit Dimethylformamid-Derivaten wie z.B. Bis(dimethylamino)-tert-butoxymethan, Dialkoxy-dialkylamino-methanen hergestellt werden.

Die Verbindungen der allgemeinen Formel (XI) können hergestellt werden, indem man die Verbindungen der allgemeinen Formel (XV) 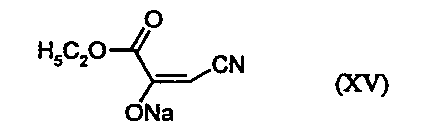 mit den Verbindungen der allgemeinen Formel (XVI)

A―CH₂-NH-NH₂ (XVI)

in Ethern, vorzugsweise Dioxan und Trifluoressigsäure in die Verbindungen der allgemeinen Formel (XVII) 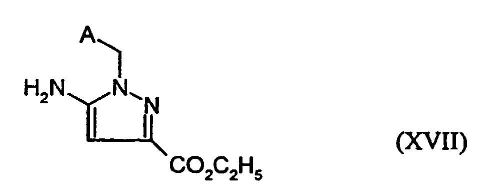 überführt,
anschließend durch Umsetzung mit den Verbindungen der allgemeinen Formel (XVIII)

Z-CH=CH-CHO (XVIII)

worin
- Z: die oben angegebenen Bedeutungen, insbesondere -N(CH₃)₂, haben kann,
in inerten Lösemitteln, vorzugsweise Dioxan, die Verbindungen der allgemeinen Formel (XIX) 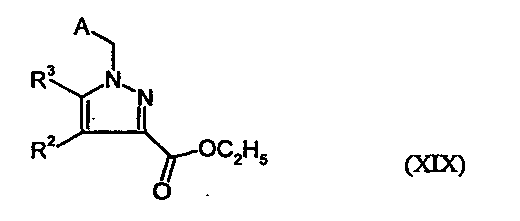 herstellt und in einem letzten Schritt mit Ammoniak in Methanol versetzt.

Anstelle des Natriumsalzes des Enolates (XV) können auch Enolether, Ketone oder Enamine eingesetzt werden.

Gegebenenfalls kann die Umsetzung der Verbindungen der allgemeinen Formeln (XV) und (XVI) zu (XVII) auch über Zwischenverbindungen der Formeln (A) und (B), 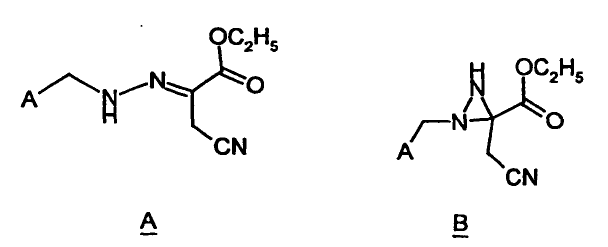 bei Raumtemperatur erfolgen.

Die Verbindungen der allgemeinen Formel (X) können beispielsweise hergestellt werden, indem man die Verbindungen der Formel (XX) oder (XXa)

[Alk₂N]₂―CH―OAlk' (XX)

Alk₂N―CH―[OAlk']₂ (XXa)

worin
- Alk und Alk': gleich oder verschieden sind und für geradkettiges oder verzweigtes Alkyl mit bis zu 5 Kohlenstoffatomen stehen,
mit Verbindungen der Formel (XXI)

R^{1'}-CH₂-CN (XXI)

worin
- R^{1'}: für den oben unter R¹ aufgeführten Cycloalkylrest steht
umsetzt.

Die Verbindungen der allgemeinen Formeln (XX), (XXa) und (XXI) sind bekannt oder nach üblichen Methoden herstellbar.

Die Verbindungen der allgemeinen Formeln (XII), (XIII), (XIV), (XV), (XV), (XVII), (XVIII) und (XIX) sind teilweise neu und können wie oben beschrieben hergestellt werden.

Der Pyrimidinrest kann auch mit Hilfe des Reagenz der Formel (Xa) aufgebaut werden, das beispielsweise folgendermaßen zugänglich ist:

### Verbindungen der allgemeinen Formel

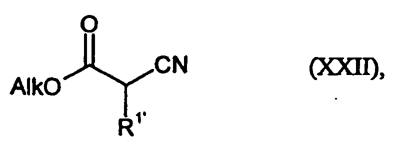

worin
- R^{1'}: die oben angegebene Bedeutung hat und Alk für einen Alkylrest mit bis zu 4 Kohlenstoffatomen steht,
werden mittels Ammoniak in geeigneten Lösungsmitteln, vorzugsweise Alkoholen wie Methanol, bei Temperaturen von 0°C bis 40°C, vorzugsweise Raumtemperatur, in Verbindungen der allgemeinen Formel (XXIII) 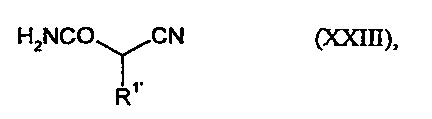 überführt, worin R¹' die oben angegebene Bedeutung hat,
und diese anschließend nach übliche Methoden durch wasserentziehende Mittel, wie beispielsweise das Burgess-Reagenz, POCl₃, P₂O₅, SOCl₂, Trifluoressigsäureanhydrid/Pyridin, umgesetzt.

Bei Verwendung des Burgess-Reagenz wird die Reaktion vorzugsweise in inerten Lösemitteln wie Ethern oder chlorierten Kohlenwasserstoffen durchgeführt. Als Beispiele seien Dichlormethan und Tetrahydrofuran genannt. Vorzugsweise wird ein 1:2-Gemisch der vorgenannten Lösemittel verwendet. Die Reaktion wird bei Temperaturen von 0°C bis 40°C, vorzugsweise bei Raumtemperatur, durchgeführt.

Die Verbindungen der Formel (XXII) sind bekannt und/oder auf einfache und dem Fachmann geläufige Weise zugänglich.

Die Verbindungen der Formel (X) unterliegen zum Teil der Keto-Enol-Tautomerie, z.B.:

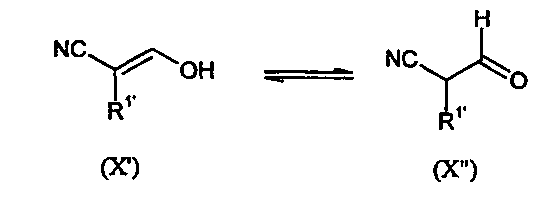

Der Pyrimidinrest kann auch mit Hilfe des Reagenz der Formel (Xa) aufgebaut werden, das beispielsweise folgendermaßen zugänglich ist:

### Verbindungen der allgemeinen Formel (XXIIa)

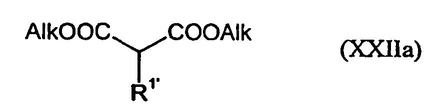

worin
- R^{1'}: die oben angegebene Bedeutung hat und
- Alk: für einen Alkylrest mit bis zu 4 Kohlenstoffatomen steht
werden mittels Ammoniak in geeigneten Lösungsmitteln, vorzugsweise Alkoholen wie Methanol, bei Temperaturen von 0°C bis 40°C, vorzugsweise Raumtemperatur, in Verbindungen der allgemeinen Formel (XXIIIa) 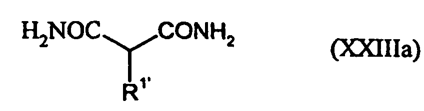 überführt, worin R^{1'} die oben angegebene Bedeutung hat, und diese anschließend nach übliche Methoden durch wasserentziehende Mittel, wie beispielsweise das Burgess-Reagenz, POCl₃, P₂O₅, SOCl₂, Trifluoressigsäureanhydrid/Pyridin, umgesetzt.

Bei Verwendung des Burgess-Reagenz wird die Reaktion vorzugsweise in inerten Lösesmitteln wie Ethern oder chlorierten Kohlenwasserstoffen durchgeführt. Als Beispiele seien Dichlormethan und Tetrahydrofuran genannt. Vorzugsweise wird ein 1:2-Gemisch der vorgenannten Lösemittel verwendet. Die Reaktion wird bei Temperaturen von 0°C bis 40°C, vorzugsweise bei Raumtemperatur, durchgeführt.

Die Verbindungen der Formel (XXIIa) sind bekannt und/oder auf einfache und dem Fachmann geläufige Weise zugänglich.

Für den Fall, daß typische Schutzgruppen im Rahmen von Derivatisierungsreaktionen eingesetzt werden, erfolgt deren Abspaltung im allgemeinen in einem der oben aufgeführten Alkohole und/oder THF oder Aceton, vorzugsweise Methanol / THF in Anwesenheit von Salzsäure oder Trifluoressigsäure oder Toluolsulfonsäure in einem Temperaturbereich von 0°C bis 70°C, vorzugsweise bei Raumtemperatur und Normaldruck.

Die erfindungsgemäßen Verbindungen der allgemeinen Formel (I) zeigen ein nicht vorhersehbares, wertvolles pbarmakologisches Wirkspektrum.

Die erfindungsgemäßen Verbindungen der allgemeinen Formel (I) führen zu einer Gefäßrelaxation, Thrombozytenaggregationshemmung und zu einer Blutdrucksenkung sowie zu einer Steigerung des koronaren Blutflusses. Diese Wirkungen sind über eine direkte Stimulation der löslichen Guanylatzyklase und einem intrazellulären cGMP-Anstieg vermittelt. Außerdem verstärken die erfindungsgemäßen Verbindungen der allgemeinen Formel (I) die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, asthmatischen Erkrankungen und Krankheiten des Urogenitalsystems wie beispielsweise Prostatahypertrophie, erektile Dysfunktion, weibliche sexuelle Dysfunktion und Inkontinenz eingesetzt werden.

Die in der vorliegenden Erfindung beschriebenen Verbindungen der allgemeinen Formel (I) stellen auch Wirkstoffe zur Bekämpfung von Krankheiten im Zentralnervensystem dar, die durch Störungen des NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuß- und Suchtmittelaufnahme.

Weiterhin eignen sich die Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Him-Traumas. Ebenso können die erfindungsgemäßen Verbindungen der allgemeinen Formel (I) zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Darüber hinaus umfaßt die Erfindung die Kombination der erfindungsgemäßen Verbindungen der allgemeinen Formel (I) mit organischen Nitraten und NO-Donatoren.

Organische Nitrate und NO-Donatoren im Rahmen der Erfindung sind im allgemeinen Substanzen, die über die Freisetzung von NO bzw. NO-Species ihre therapeutische Wirkung entfalten. Bevorzugt sind Natriumnitroprussid, Nitroglycerin, Isosorbiddinitrat, Isosorbidmononitrat, Molsidomin und SIN-1.

Außerdem umfaßt die Erfindung die Kombination mit Verbindungen, die den Abbau von cyclischem Guanosinmonophosphat (cGMP) inhibieren. Dies sind insbesondere Inhibitoren der Phosphodiesterasen 1, 2 und 5; Nomenklatur nach Beavo und Reifsnyder (1990) TiPS 11 S. 150 bis 155. Durch diese Inhibitoren wird die Wirkung der erfindungsgemäßen Verbindung potenziert und der gewünschte pharmakologische Effekt gesteigert.

Zur Feststellung der kardiovaskulären Wirkungen wurden folgende Untersuchungen durchgeführt: In in vitro-Untersuchungen am isolierten Enzym und an Zellen vaskulären Ursprungs wurde der Einfluß auf die Guanylatzyklase-abhängige cGMP-Bildung mit und ohne NO-Donor geprüft. Die antiaggregatorischen Eigenschaften wurden an mit Kollagen stimulierten menschlichen Thrombozyten gezeigt. Die gefäßrelaxierende Wirkung wurde an mit Phenylephrin vorkontrahierten Kaninchenaortenringen bestimmt. Die blutdrucksenkenden Wirkungen wurden an narkotisierten und wachen Ratten untersucht.

### Stimulation der rekombinanten löslichen Guanylatzyklase in vitro

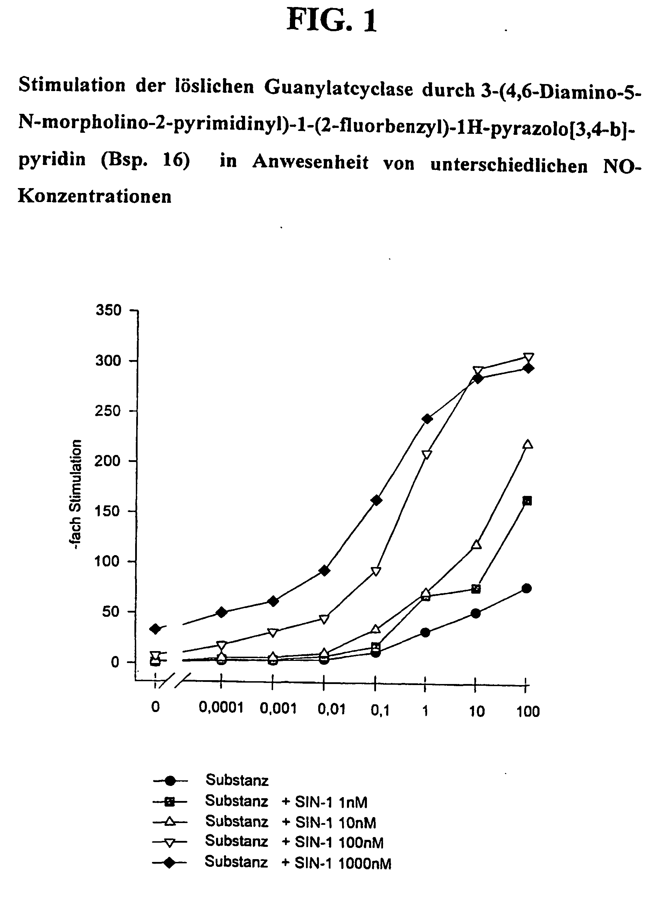

Die Untersuchungen zur Stimulation der rekombinanten löslichen Guanylatzyklase und die erfindungsgemäßen Verbindungen mit und ohne NO-Donor wurden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch: Purified soluble guanylyl cyclase expressed in a baculovirus/Sf9 system: stimulation by YC-1, nitric oxide, and carbon oxide. J. Mol. Med. 77: 14-23 (1999). Die Ergebnisse sind in Fig. 1 gezeigt.

### Stimulation der löslichen Guanylatzyklase in primären Endothelzellen

Primäre Endothelzellen wurden aus Schweineaorten durch Behandlung mit Kollagenase-Lsg. isoliert. Anschließend wurden die Zellen in Kulturmedium bei 37°C /5% CO₂ bis zum Erreichen der Konfluenz kultiviert. Für die Untersuchungen wurden die Zellen passagiert, in 24-Loch Zellkulturplatten ausgesät und bis zum Erreichen der Konfluenz subkultiviert (~ 2 x 10⁵ Zellen / Vertiefung). Zur Stimulation der endothelialen Guanylatzyklase wurde das Kulturmedium abgesaugt und die Zellen einmal mit Ringerlösung gewaschen. Nach Entfernen der Ringerlösung wurden die Zellen in Stimulationspuffer mit oder ohne NO-Donor (Natrium-Nitroprussid, SNP oder DEA/NO 1 µM) 10 Minuten bei 37°C / 5% CO₂ inkubiert. Im Anschluß daran wurden die Testsubstanzen (Endkonzentration 1 µM) zu den Zellen pipettiert und weitere 10 Minuten inkubiert. Nach Ende der Inkubationszeit wurde die Pufferlösung abgesaugt und 4°C kalter Stoppuffer zu den Zellen gegeben. Die Zellen wurden dann 16 Stunden lang bei -20°C lysiert. Anschließend wurden die das intrazelluläre cGMP enthaltenden Überstände abgenommen und die cGMP-Konzentrationen durch das cGMP-SPA-System (Amersham Buchler, Braunschweig) bestimmt. Die Ergebnisse sind in den nachstehenden Tabellen 1 und 2 aufgeführt:

**Tabelle 1:**

| **Stimulation der löslichen Guanylatzyklase in primären Endothelzellen** | |
|---|---|
| Beispiel Nr. | Steigerung der cGMP-Konzentration (%) |
| 1 | >1000 |
| 2 | >1000 |
| 3 | >1000 |
| 6 | 600 |
| 13 | >1000 |
| 14 | >1000 |

**Tabelle 2:**

| **Stimulation der löslichen Guanylatzyklase in primären Endothelzellen durch 3-(4,6-Diamino-5-N-morpholinopyrimidin-2-yl)-1-(2-fluorbenzyl)-1H-pyrazolo [3,4-b]-pyridin (Bsp. 16)** | |
|---|---|
| Bsp. 16 (µM) | cGMP (pmol/well) |
| 0 | 1,7 |
| 0,1 | 5,1 |
| 0,3 | 13,2 |
| 1,0 | 20,8 |
| 3,0 | 34,5 |
| 10 | 47,7 |

### Gefäßrelaxierende Wirkung in vitro

Kaninchen werden durch Nackenschlag betäubt und entblutet. Die Aorta wird entnommen, von anhaftendem Gewebe befreit, in 1,5 mm breite Ringe geteilt und einzeln unter einer Vorspannung in 5 ml-Organbäder mit 37°C warmer, carbogenbegaster Krebs-Henseleit-Lösung folgender Zusammensetzung (mM) gebracht: NaCl: 119; KCl: 4,8; CaCl₂ x 2 H₂O: 1; MgSO₄ x 7 H₂O; 1,4; KH₂PO₄: 1,2; NaHCO₃:25; Glucose: 10. Die Kontraktionskraft wird mit Statham UC2-Zellen erfaßt, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung untersucht und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀). Das Standardapplikationsvolumen beträgt 5 µl, der DMSO-Anteil in der Badlösung entspricht 0,1 %. Die Ergebnisse sind nachstehend in Tabelle 3 aufgeführt:

**Tabelle 3:**

| **Gefäßrelaxierende Wirkung in vitro** | |
|---|---|
| Beispiel Nr. | IC₅₀ [µM] |
| 1 | 0,23 |
| 3 | 0,38 |
| 3 | 0,4 |
| 5 | 0,24 |
| 7 | 3,4 |
| 13 | 0,41 |
| 16 | 0,2 |
| 21 | 0,67 |
| 22 | 0,68 |
| 23 | 0,54 |
| 24 | 0,35 |
| 25 | 0,79 |
| 26 | 1 |
| 27 | 0,18 |
| 28 | 0,22 |
| 31 | 0,53 |
| 32 | 0,58 |
| 33 | 0,62 |
| 35 | 1,8 |
| 71 | 0,7 |
| 73 | 0,69 |
| 77 | 0,76 |
| 78 | 9,5 |
| 79 | 4,1 |

### Blutdruckmessungen an narkotisierten Ratten

Männliche Wistar-Ratten mit einem Körpergewicht von 300 - 350 g werden mit Thiopental (100 mg/kg i.p.) anästhesiert. Nach Tracheotomie wird in die Femoralarterie ein Katheter zur Blutdruckmessung eingeführt. Die zu prüfenden Substanzen werden in Transcutol, Cremophor EL, H₂O (10%/20%/70%) in einem Volumen von 1 ml/kg oral verabreicht Die Ergebnisse sind in der nachstehenden Tabelle 4 aufgeführt.

**Tabelle 4**

| Bsp.-Nr. | Dosis (mg/kg p.o.) | max. Blutdrucksenkung (mm Hg) |
|---|---|---|
| 16 | 0.3 | 21 |
| 16 | 1.0 | 35 |

### Wirkung auf den mittleren Blutdruck von wachen, spontan hypertensiven Ratten

Kontinuierliche Blutdruckmessungen über 24 Stunden wurden an spontan hypertonen 200-250g schweren sich frei bewegenden weiblichen Ratten (MOL:SPRD) durchgeführt. Dazu waren den Tieren chronisch Druckaufnehmer (Data Sciences Inc., St. Paul, MN, USA) in die absteigende Bauchaorta unterhalb der Nierenarterie implantiert und der damit verbundene Sender in der Bauchhöhle fixiert worden.

Die Tiere wurden einzeln in Type III Käfigen, die auf den individuellen Empfängerstationen positioniert waren, gehalten und waren an einem 12-Stunden Hell/Dunkel-Rhythmus angepaßt. Wasser und Futter standen frei zur Verfügung.

Zur Datenerfassung wurde der Blutdruck jeder Ratte alle 5 Minuten für 10 Sekunden registriert. Die Meßpunkte wurden jeweils für eine Periode von 15 Minuten zusammengefaßt und der Mittelwert aus diesen Werten berechnet.

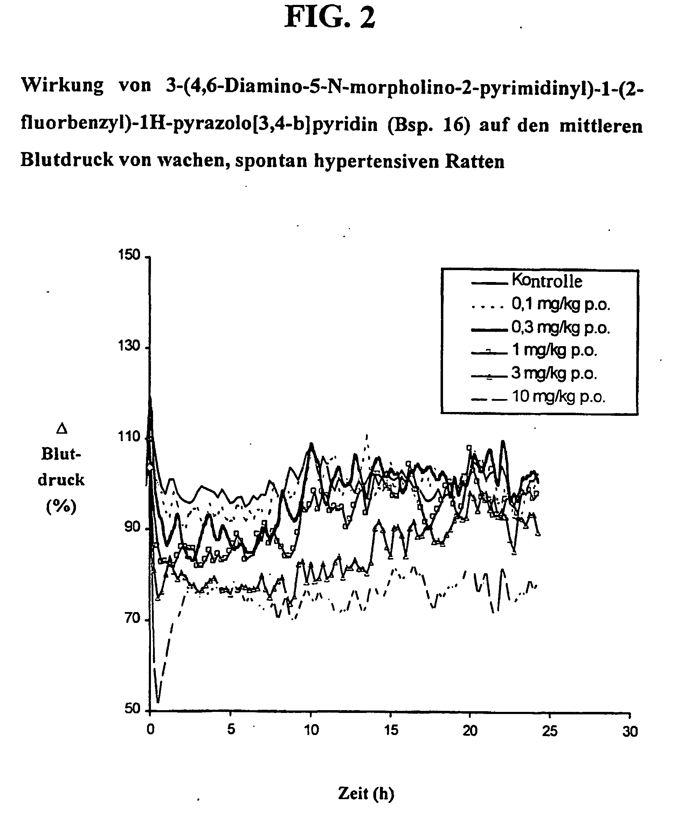

Die Prüfverbindungen wurden in einer Mischung aus Transcutol (10%), Cremophor (20%), H₂O (70%) gelöst und mittels Schlundsonde in einem Volumen von 2 ml/kg Körpergewicht oral verabreicht. Die Prüfdosen lagen zwischen 0,3 -30 mg/kg Körpergewicht. Die Ergebnisse sind in der beiliegenden Fig. 2 gezeigt

### Thrombozytenaggregationshemmung in vitro

Zur Bestimmung der Thrombozytenaggregation wurde Blut von gesunden Probanden beiderlei Geschlechts verwendet. Als Antikoagulans wurde einem Teil 3,8%iger Natriumzitratlösung 9 Teile Blut zugemischt. Das Blut wurde mit 900U/min für 20min zentrifugiert. Der pH Wert des gewonnenen plättchenreichen Plasmas wurde mit ACD-Lösung (Natriumcitrat/Citronensäure/Glucose) auf pH 6,5 eingestellt. Die Thrombozyten wurden anschließend abzentrifugiert und in Puffer aufgenommen und wiederum abzentrifugiert. Der Thrombozytenniederschlag wurde in Puffer aufgenommen und zusätzlich mit 2 mmol/l CaCl₂ versetzt.

Für die Aggregationsmessungen wurden Aliquots der Thrombozytensuspension mit der Prüfsubstanz 10 min bei 37°C inkubiert. Anschließend wurde die Aggregation durch Zugabe von Kollagen in einem Aggregometer ausgelöst und mittels der turbidometrischen Methode nach Born (Born, G.V.R., J.Physiol. (London), 168, 178-195, 1963) bei 37°C bestimmt. Die Ergebnisse sind nachstehend in Tabelle 5 aufgeführt.

**Tabelle 5**

| Bsp.-Nr. | IC₅₀ (µM) |
|---|---|
| 16 | 0.06 |

### Messung der erektionsfördernden Wirksamkeit von Guanylatcyclase-Stimulatoren

Für das Zustandekommen einer vollständigen und anhaltenden Erektion müssen die cavernösen Arterien und die gesamte Schwellkörperarchitektur, die aus einem Netzwerk von glatten Muskelzellen und kollagenem Bindegewebe gebildet wird, maximal dilatieren, damit sich der Corpus cavernosum vollständig mit Blut füllen kann (Anderson K.-E. and Wagner G.,"Physiology of Penile Erection.". *Physiological Reviews 75, 191-236 (1995);* Meinhardt W. Kropmann RF, Vermeig P, Lycclama a Nigelholt and Zwartendijk J. "The Influence of Medication on Erectile dysfunction." *Int. J. of Impotence Res. 9, 17-26 (1997).* Die Relaxation der glatten Muskulatur wird durch NO vermittelt, das bei sexueller Stimulation von nicht adreneregen, nicht cholinergen Nervenfasem in den Endothelzellen der Blutgefäße des Corpus cavemosum freigesetzt wird. NO aktiviert die Guanylatcyclase, der daraus resultierende Anstieg des cGMP führt zur Dilatation der glatten Muskulatur des Corpus cavemosum und damit zu einer Erektion. Zur Prüfung der Wirksamkeit der erfindungsgemäßen Substanzen wurden wache Kaninchen eingesetzt. Die Spezies Kaninchen wurde gewählt, da die Neurophysiologie, die Haemodynamik und die Steuerung der Kontraktion und der Relaxation der glatten Muskulatur des Schwellkörpers bei Kanichen und Mensch recht ähnlich sind (Meyer MF, Taher H., Krah H., Staubesand J., Becker AJ, Kircher M., Mayer B., Jonas U., Forsmann WG., Stief Ch.G. "Intracarvenous Application of SIN-1 in Rabbit and Man: Functional and Toxcological Results." *Annals Urol. 27, 179-182 (1993);* Taub HC, Lerner, SE, Melman A, Christ GJ "Relationship between contraction and relaxation in human and rabbit corpus cavernosum." *Urology 42, 698-671, (1993).*

### Methode:

Adulte, männliche Chinchilla-Kaninchen mit einem Gewicht von 3 -5 kg werden nach Lieferung mehrere Tage in Einzelhaltung adaptiert. Sie haben freien Zugang zu Wasser und können zwei Stunden pro Tag Futter zu sich nehmen. Die Tiere werden in einem 10/14 Stunden Tag-Nacht Rhythmus gehalten (Licht an, ab 8.00 Uhr), die Raumtemperatur beträgt 22 -24 °C.

Die Tiere werden direkt vor Versuchsbeginn gewogen. Für die intravenöse Administration wurden die erfindungsgemäßen Substanzen in einem Gemisch von Transcutol (GATTEFOSSE GmbH) verdünnt mit 20% Cremophor (BASF) und Wasser im Verhältniss von 3/7 gelöst. Natriumnitroprussid wurde in 0,9% NaCl gelöst. Die Substanzen wurden in den in der Tabelle angegeben Dosierungen in einem Volumen von 0,5 ml/kg die Ohrvene injeziert. Für die orale Gabe wurden die Testsubstanzen in einer Mischung aus Glycerin: Wasser: Polyethylenglykol 6:10:9,69 gelöst und in den in der Tabelle angegebenen Dosierungen in einem Volumen von 1 ml/kg mit der Schlundsonde appliziert.

DieWirkung von Guanylatcyclasestimulatoren wird durch NO-Donatoren verstärkt. Dies wurde mit der zusätzlichen Gabe von Natriumnitroprussid demonstriert.

Das Natriumnitroprussid wurde in einer Dosierung von 0,2 mg/kg gleichzeitig mit der erfindungsgemäßen Substanz in die Ohrvene injeziert. Wurde die erfindungsgemäße Substanz oral gegeben so wurde diesen Tieren das Natriumnitroprussid 30 min. nach der oralen Gabe in die Ohrvene injiziert. Entsprechende Kontrollen mit dem Lösungsmittel und mit Natriumnitroprussid alleine wurden durchgeführt.

Unter Ruhebedingungen ist der Kaninchenpenis in der Schamregion nicht sichtbar und von der Penishaut vollständig bedeckt. Die Erektion wird gewertet, in dem man die Länge des hervortretenden Penis mit einer Schiebelehre misst. Die Messung wird 5, 10, 15, 30, 45, 60min. 120 und 180 min. nach Verabreichung der Substanz durchgefürt. Die Wirkung wird als Produkt der Länge des nicht von Fell bedeckten Penis in [mm]und der Zeit die die Erektion anhält in [min.] berechnet.

Die intravenöse Injektion von Natriumnitroprussid bewirkt eine ca 10 min. anhaltende Erektion (110 [mm x min.]).

Zur vorliegenden Erfindung gehören pharmazeutische Zubereitungen, die neben nichttoxischen, inerten pharmazeutisch geeigneten Trägerstofien die erfindungsgemäßen Verbindungen der allgemeinen Formel (I) enthält sowie Verfahren zur Herstellung dieser Zubereitungen.

Die Wirkstoffe können gegebenenfalls in einem oder mehreren der oben angegebenen Trägerstoffe auch in mikroverkapselter Form vorliegen.

Die therapeutisch wirksamen Verbindungen der allgemeinen Formel (I) sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen der allgemeinen Formel (I) auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente.

### Beispiele

### Abkürzungen:

- RT:: Raumtemperatur
- EE:: Essigsäureethylester
- MCPBA:: m-Chlorperoxybenzoesäure
- BABA:: n-Butylacetat/n-Butanol/Eisessig/Phosphatpuffer pH 6 (50:9:25:15; org. Phase)

### Laufmittel für die Dünnschichtchromatographie:

- T1 E1:: Toluol - Essigsäureethylester (1:1)
- T1 EtOH1:: Toluol - Methanol (1:1)
- C1 E1 :: Cyclohexan - Essigsäureethylester (1:1)
- C1 E2:: Cyclohexan - Essigsäureethylester (1:2)

### Ausgangsverbindungen:

### Allgemeine Vorschrift zur Herstellung von 2-substituierten 3-Dimethylaminoacrylnitrilen

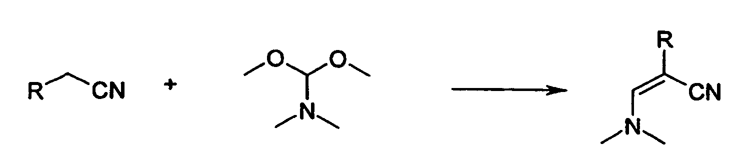

Zu einer Lösung von 5.95 g (50.0 mmol) N,N-Dimethylformamid-dimethylacetal in 25 ml abs. Methanol werden unter Wasserkühlung 50.0 mmol 2-substituiertes Acetonitril-Derivat gegeben und 1 h bei Raumtemperatur gerührt.
- Sulfone:: Der Niederschlag wird abgesaugt und im Hochvakuum getrocknet
- Phosphonsäure-Ester:: Die Lösung wird zunächst bei 40°C und 20 mbar am Rotationsverdampfer, dann bei Raumtemperatur am Hochvakuum vom Methanol befreit.

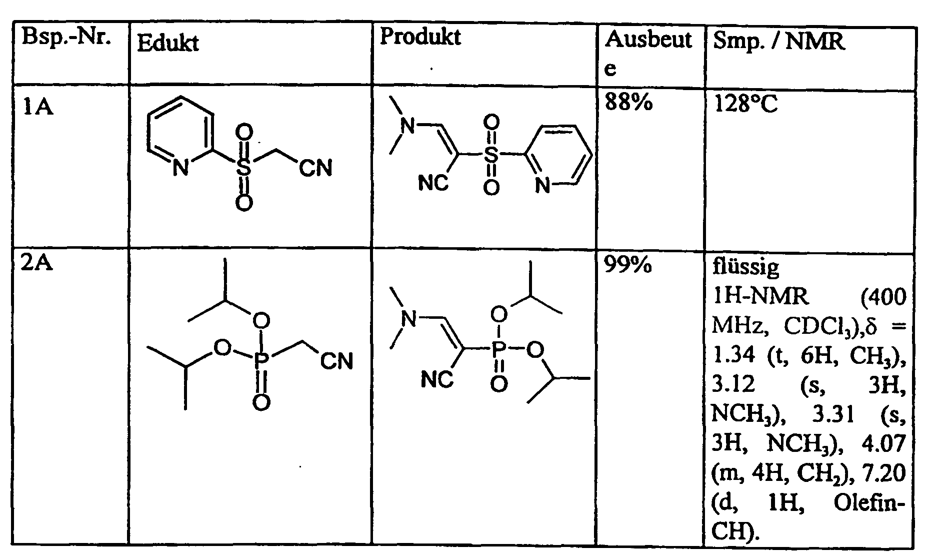

### Beispiel 3A

### 3-(Dimethylamino)-2-N-morpholinoacrylnitril

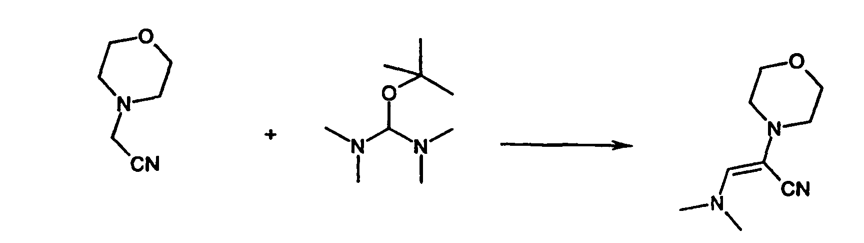

8.13 g (64.5 mmol) Morpholinoacetonitril und 13.3 ml (64.5 mmol) tert-Butoxy-bis(dimethylamino)methan wurden über Nacht bei 80 °C gerührt. Die auf Raumtemperatur abgekühlte Mischung wird am Rotationsverdampfer eingeengt und anschließend im Vakuum destilliert.
- Ausbeute:: 11.0 g (94%) (cis- und trans-Isomer)
- Siedepunkt:: 119°C / 0.008 mbar

### Beispiel 4A

### 3-(Dimethylamino)-2-N-thiomorpholinoacrylnitril

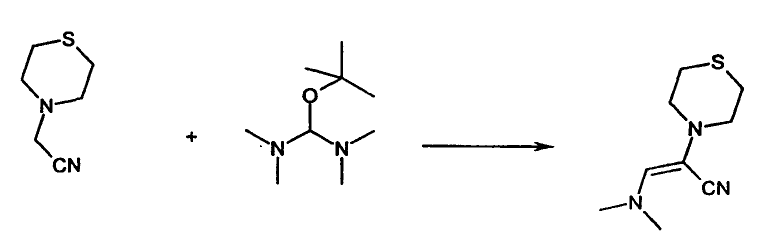

6.65 g (46.8 mmol) N-Thiomorpholinoacetonitril (Wise,L.D. et al., J.Med.Chem., 17, 1974, 1232-1234) und 9.70 ml (47.0 mmol) tert-Butoxy-bis(dimethylamino)methan wurden über Nacht bei 80 °C gerührt. Die auf Raumtemperatur abgekühlte Mischung wird am Rotationsverdampfer eingeengt und anschließend im Vakuum destilliert.
- Ausbeute:: 9.98 g (88% bzgl. Gehalt an Reinsubstanz, cis- und trans-Isomeren)
- Siedepunkt:: 96°C / 0.008 mbar

### Beispiel 5A

### 3-Dimethylamino-2-methylsulfonylacrylsäureethylester.

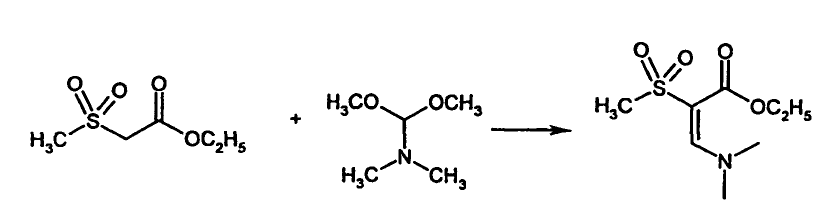

6.65 g (40 mmol) Methanesulfonylessigsäureethylester und 5.72 g (48 mmol) N,N-Dimethylformamiddimethylacetal werden zusammengegeben und auf 85°C über Nacht erhitzt. Die Lösung wird einrotiert und der Feststoff mit Cyclohexan zerkleinert und abgesaugt.
Ausbeute: 8.36 g (94.5 % d. Th.)

### Beispiel 6 A

### 2-(4-Methylpiperazino)malonsäurediamid

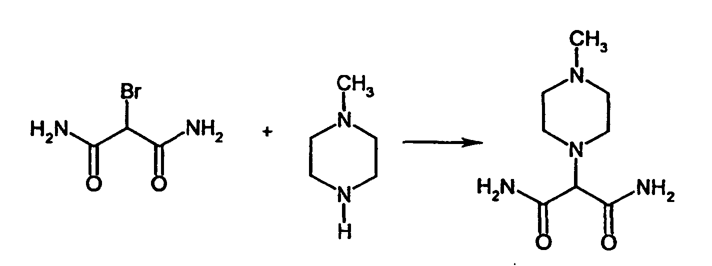

1.00 g (5.52 mmol) 2-Brommalonsäurediamid (Darstellung analog Backes, West und Whiteley, J. Chem. Soc. **1921,** *119,* 359), 0.61 g (6.10 mmol) N-Methylpiperazin und 1.15 g (8.29 mmol) Kaliumcarbonat werden in 10 mL Acetonitril zusammengegeben und über Nacht auf 50°C erhitzt. Die Mischung wird abfiltriert und das Feststoff wird mit kochendem Ethanol digeriert und abgesaugt. Das Filtrat wird einrotiert und das Produkt roh weiter umgesetzt.
Ausbeute: 1.14 g (Rohausbeute).
Rf (SiO₂, BABA): 0.06

### Beispiel 7A

### 2-(4-Acetylpiperazino)malonsäurediamid

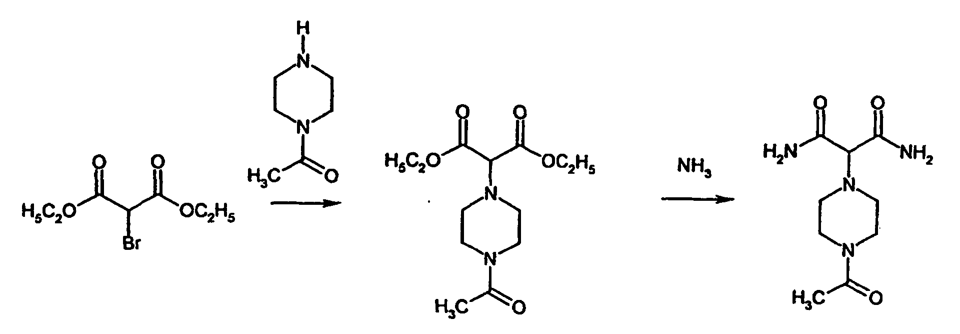

6.16 g (25.8 mmol) 2-Brommalonsäurediethylester, 3.63 g (28.3 mmol) N-Acetylpiperazin und 5.34 g (38.6 mmol) Kaliumcarbonat werden in 100 mL Acetonitril zusammengegeben und 28 Stunden auf 50°C erhitzt. Das Reaktionsgemisch wird abgekühlt, in 50 mL Essigsäureethylester aufgenommen und mit Wasser gewaschen. Die organische Phase wird über Magnesiumsulfat getrocknet und einrotiert. Ausbeute: 8.57 g. 7 g vom Produkt wird roh in 70 mL Ammoniaklösung in Methanol gelöst und 90 Stunden bei Raumtemperatur gerührt. Das Feststoff wird abgesaugt, mit kaltem Methanol gewaschen und getrocknet.
Ausbeute: 2.76 g (49.6% d. Th.).

### Beispiel 8 A

### 2-(4-Methylpiperazin)malondinitril

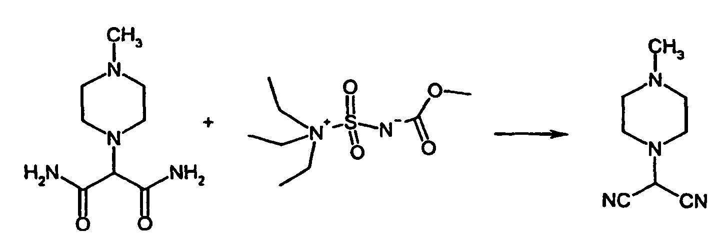

441 mg (2.04 mmol) 2-(4-Methylpiperazin)malonsäurediamid aus Beispiel 6A werden in 20 mL Tetrahydrofuran:Dichlormethan (3:1) gelöst. In drei gleichen Portionen im Abstand von 30 Minuten wird insgesamt 1.70 g (7.15 mmol) des Burgess Reagenzes zugegeben. Nach weiteren dreißig Minuten wird das Reaktionsgemisch direkt an Kieselgel mit Essigester als Eluent chromatographiert.
Ausbeute: 233 mg (69.4 % d. Th.).
Rf (SiO₂, EE): 0.22

Analog zu den Beispielen 6A bis 8A wurden dargestellt:

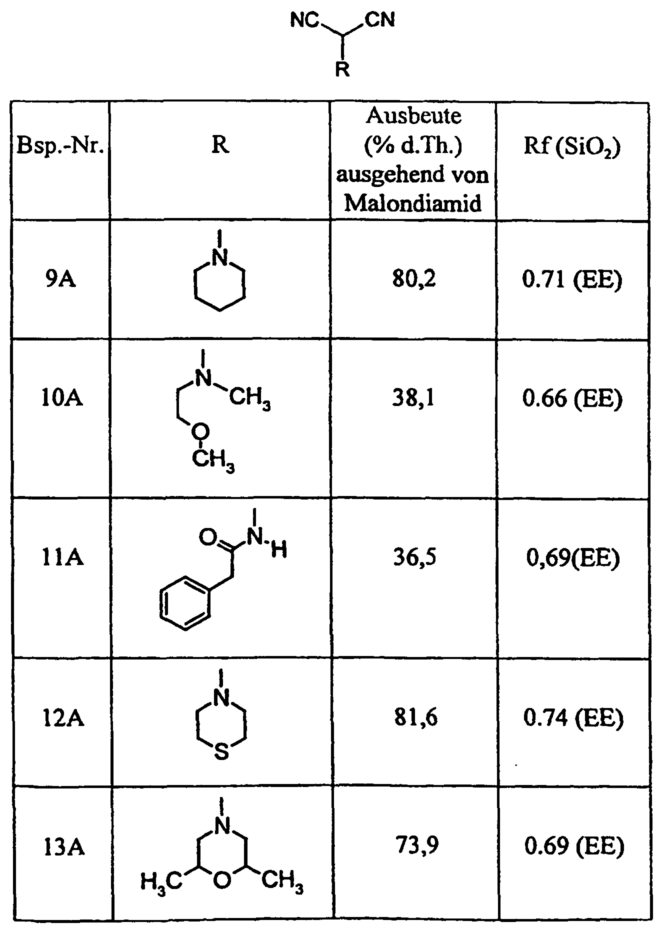

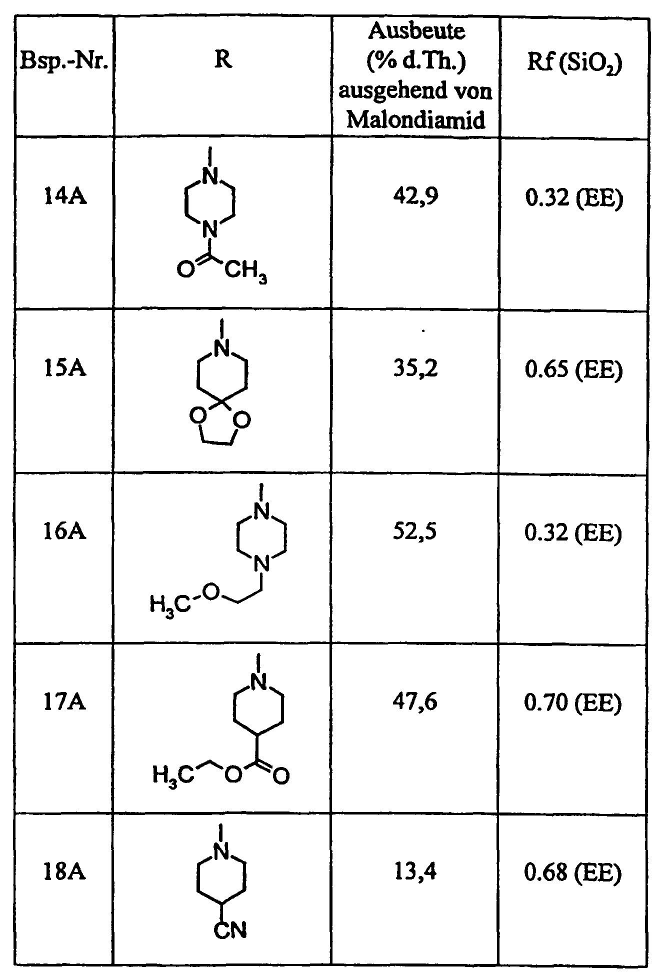

Die Darstellung von 2-N-Morpholinomalondinitril, 2-(N,N-Dimethylamino)-malondinitril und 2-(N,N-Diethylamino)malondinitril wird nach H. Gold und O. Bayer, *Chem. Ber.* **1961**, *94*, 2594, durchgeführt.

Die Darstellung von 2-(1,3-Thiazol-2-yl)-malononitril wird nach Yamanaka, H.; Ohba, S.; Sakamoto, T. *Heterocycles*, **1990**, *31*, 1115, durchgeführt.

### Beispiel 19 A

### 2-(5-Methyl-1,3,4-thiadiazol-2-yl)-malononitril

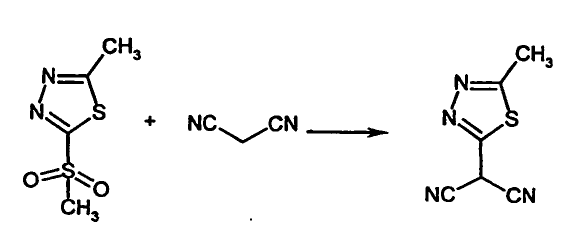

400 mg (10.0 mmol) Natriumhydrid (60% Suspension in Öl) werden unter Argon in 40 mL THF bei Raumtemperatur suspendiert. Eine Lösung von 661 mg (10.0 mmol) Malondinitril in 10 mL THF wird tropfenweise zugegeben und die Reaktionsmischung wird 15 min. gerührt. Eine Lösung von 891 mg (5.0 mmol) 2-Methyl-5-methylsulfony-1,3,4-thiadiazol in 10ml THF wird tropfenweise zugegeben. Die Reaktionsmischung wird auf 50°C erhitzt und über Nacht gerührt, abgekühlt und einrotiert. Der Rückstand wird in Wasser gelöst und mit HCl auf pH=3 einstellen. Ein brauner Feststoff fällt aus, der abfiltriert und im Vakuum getrocknet wird.
Ausbeute: 714 mg (87.0 % d. Th.).
Smp: 210°C (Z)

### Beispiel 20A

### 5-Amino-1-(2-fluorbenzyl)-1H-pyrazol-3-carbonsäureethylester

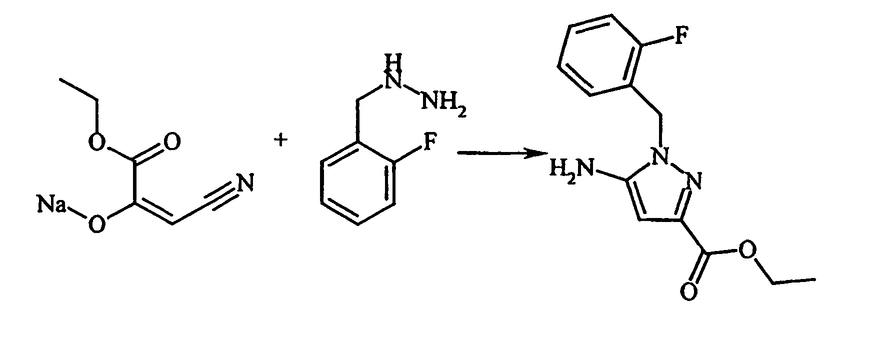

100 g (0.613 mol) Natriumsalz des Cyanobrenztraubensäureethylester (Darstellung analog Borsche und Manteuffel, Liebigs Ann. 1934, *512*, 97) werden unter gutem Rühren unter Argon in 2.5 1 Dioxan bei Raumtemperatur mit 111.75 g (75 ml, 0.98 mol) Trifluoressigsäure versetzt und 10 min gerührt, wobei ein großer Teil des Eduktes in Lösung geht. Dann gibt man 85.93 g (0.613 mol) 2-Fluorbenzylhydrazin hinzu und kocht über Nacht. Nach Abkühlen werden die ausgefallenen Kristalle des Natriumtrifluoracetats abgesaugt, mit Dioxan gewaschen und die Lösung roh weiter umgesetzt.

### Beispiel 21A

### 1-(2-Fluorbenzyl)-1H-pyrazolo[3,4-b]pyridin-3-carbonsäureethylester

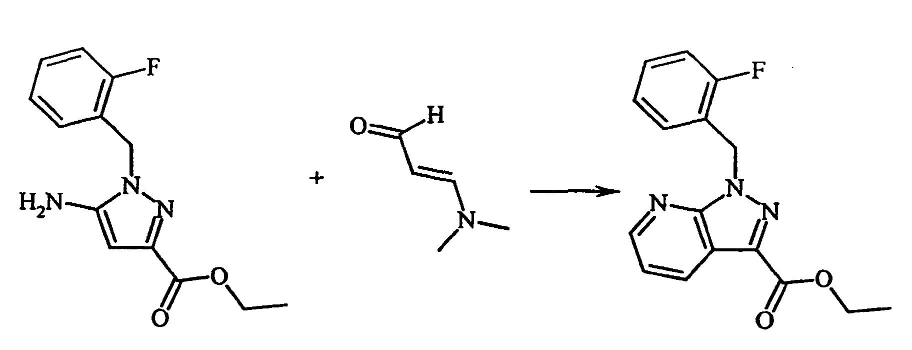

Die obige Lösung aus Beispiel 20A wird mit 61.25 ml (60.77 g, 0.613 mol) Dimethylaminoacrolein und 56.28 ml (83.88 g, 0.736 mol) Trifluoressigsäure versetzt und unter Argon 3 Tage lang gekocht. Anschließend wird das Lösungsmittel im Vakuum verdampft, der Rückstand in 21 Wasser gegeben und dreimal mit je 1 l Essigester extrahiert. Die vereinigten organischen Phasen werden mit Magnesiumsulfat getrocknet und einrotiert. Man chromatographiert auf 2.5 kg Kieselgel und eluiert mit einem Toluol / Toluol-Essigester=4:1 -Gradienten.
Ausbeute: 91.6 g (49.9 % d.Th. über zwei Stufen).
Smp. 85 °C
Rf (SiO₂, T1E1): 0.83

### Beispiel 22A

### 1-(2-Fluorbenzyl)-1H-pyrazolo[3,4-b]pyridin-3-carboxamid

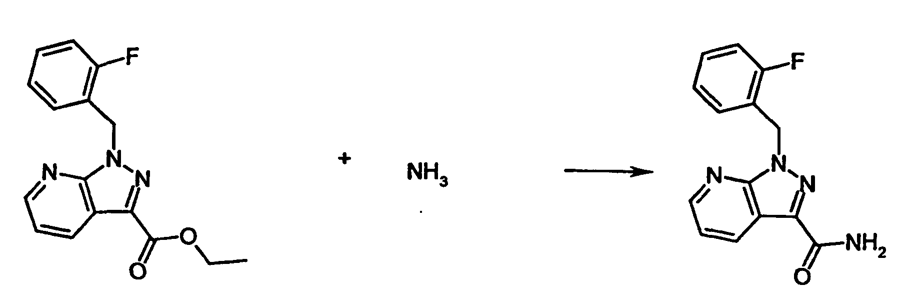

10.18 g (34 mmol) des Esters aus Beispiel 2 1 A werden in 150 ml mit Ammoniak bei 0 - 10°C gesättigtem Methanol vorgelegt. Man rührt zwei Tage bei Raumtemperatur und engt anschließend im Vakuum ein.
Rf (SiO₂, T1E1): 0.33

### Beispiel 23A

### 3-Cyano-1-(2-fluorbenzyl)-1H-pyrazolo[3,4-b]pyridin

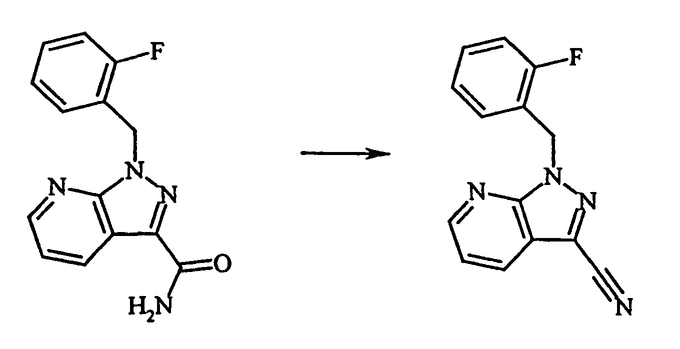

36.1 g (133 mmol) 1-(2-Fluorbenzyl)-1H-pyrazolo[3,4-b]pyridin-3-carboxamid aus Beispiel 22A werden in 330 ml THF gelöst und mit 27 g (341 mmol) Pyridin versetzt. Anschließend gibt man innerhalb von 10 min 47.76 ml (71.66 g, 341 mmol) Trifluoressigsäureanhydrid hinzu, wobei die Temperatur bis auf 40 °C ansteigt. Man rührt über Nacht bei Raumtemperatur. Anschließend wird der Ansatz in 11 Wasser gegeben und dreimal mit je 0.5 l Essigester extrahiert. Die organische Phase wird mit gesättigter Natriumhydrogencarbonatlösung und mit 1 N HCl gewaschen, mit MgSO₄ getrocknet und einrotiert.
Ausbeute: 33.7 g (100% d.Th.)
Smp: 81°C
Rf (SiO₂, T1E1): 0.74

### Beispiel 24A

### 1-(2-Fluorbenzyl)-1H-pyrazolo[3,4-b]pyridin-3-carboximidsäuremethylester

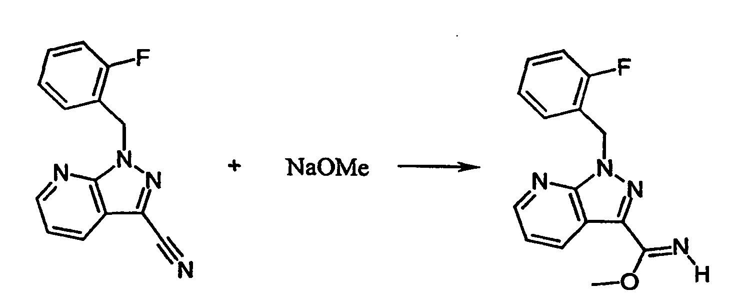

Man löst 30.37 g (562 mmol) Natriummethylat in 1.5 1 Methanol und gibt 36.45 g (144.5 mmol) 3-Cyano-1-(2-fluorbenzyl)-1H-pyrazolo[3,4-b]pyridin aus Beispiel 23A hinzu. Man rührt 2 Stunden bei Raumtemperatur und setzt die erhaltene Lösung direkt für die nächste Stufe ein.

### Beispiel 25A

### 1-(2-Fluorbenzyl)-1H-pyrazolo[3,4-b]pyridine-3-carboxamidin

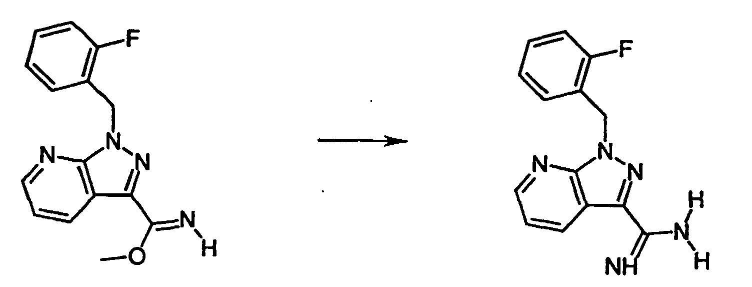

Obige Lösung von (2-Fluorbenzyl)-1H-pyrazolo[3,4-b]pyridin-3-carboximidsäure-methylester aus Beispiel 24A in Methanol wird mit 33.76 g (32.19 ml, 562 mmol) Eisessig und 9.28 g (173 mmol) Ammoniumchlorid versetzt und über Nacht unter Rückfluß gerührt. Man verdampft das Lösungsmittel im Vakuum, verreibt den Rückstand gut mit Aceton und saugt den ausgefallenen Feststoff ab. Man gibt in 21 Wasser, versetzt unter Rühren mit 31.8 g Natriumcarbonat und extrahiert dreimal mit insgesamt 11 Essigester, trocknet die organische Phase mit Magnesiumsulfat und dampft im Vakuum ein.
Ausbeute: 27.5 g (76.4 % d.Th. über zwei Stufen)
Smp.: 86 °C
Rf(SiO₂, TIEtOH1): 0.08

### Herstellungsbeispiel

### Beispiel 1

### 3-(4-Amino-5-methylsulfonylpyrimidin-2-yl)-1-(2-fluorbenzyl)-1H-pyrazolo[3,4-b]pyridin

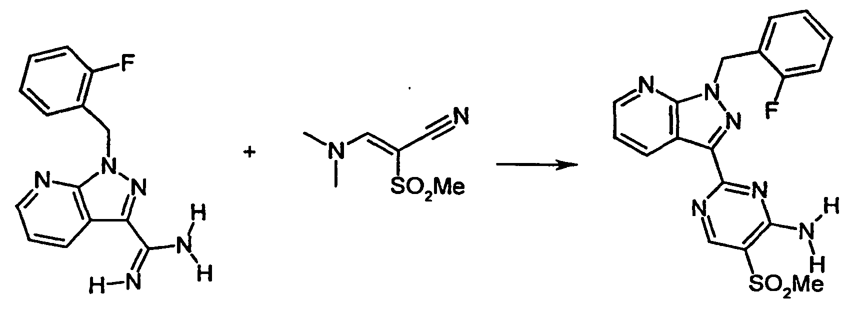

2 g (7.42 mmol) 1-(2-fluorbenzyl)-1H-pyrazolo[3,4-b]pyridin-3-carboxamidin aus Beispiel 25A, 1.39 g (7 mmol) 3-(Dimethylamino)-2-(methylsulfonyl)-2-propenenitril (das analog zu Beispiel 1A herstellbar ist), 0.79 ml (7 mmol) Piperidin und 200 ml Isoamylalkohol werden 12 h lang bei 110 °C gerührt. Nach Abkühlen werden die ausgefallenen Kristalle abgesaugt und mit Diethylether gewaschen. Man erhält 0.94 g (31.8 % d. Th.) der Titelverbindung.
Smp.: 272 °C
Rf (SiO₂, EE): 0.72

Analog wurden dargestellt:

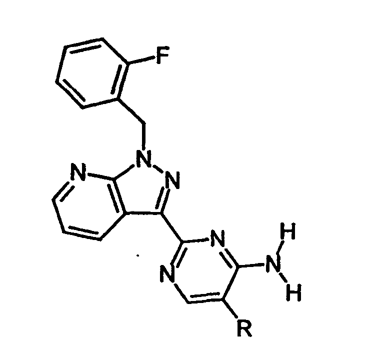

| Bsp.-Nr. | R | Smp. [°C] | Rf (SiO₂) | Ausbeute |
|---|---|---|---|---|
| 2 | CH₃CH₂CH₂SO₂- | 254 | 0.87 (EE) | 30 |
| 3 | (CH₃)₂CHSO₂- | 268 | 0.83 (EE) | 31.6 |
| 4 | (CH₃)₃C-SO₂- | >280 | 0.24 (T1E1) | 25.4 |
| 5 | [(CH₃)₂CHO]₂PO- | 190 | 0.19 (T1E1) | 10.8 |
| 6 | -CONH₂ | 215 | 0.25 (T1E1) | 9.6 |
| 7 | -SO₂-(2-thienyl) | 275 | 0.48 (T1E1) | 11.5 |
| 8 | -CH₂CF₃ | 181 | | 14.4 |
| 9 | PhSO₂- | 279 | 0.51 (T1E1) | 29.2 |
| 10 | PhSO- | 218 | 0.26 (TIE1) | 19.4 |
| 11 | -(CH₂)₅CN | 107 | 0.22 (EE) | 18 |
| 12 | -(CH₂)₇CN | 147 | 0.36 (EE) | 13.5 |
| 13 | -CH₂CH₂-CN | 201 | 0.2 (T4EtOH1) | 12 |

Die entsprechenden 3-Dimethylaminoacrylnitrile mit dem jeweiligen Substituenten R in 2-Position, welche als Ausgangsverbindungen der Beispiele 2 bis 13 mit dem Amidin 25A umzusetzen sind, können analog zu Beispiel 1A und 3A hergestellt werden.

### Beispiel 14

### 3-[5-Cyano-4-(4-methylphenyl)pyrimidin-2-yl]-1-(2-fluorbenzyl)-1H-pyrazolo[3,4-b]pyridin

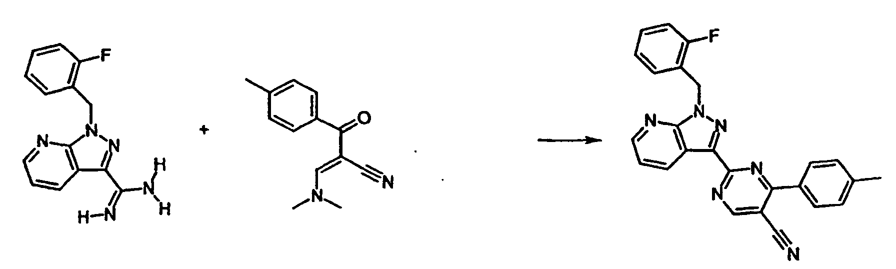

200 mg (0.74 mmol) 1-(2-fluorbenzyl)-1H-pyrazolo[3,4-b]pyridin-3-carboxamidin aus Beispiel 25A, 171 mg (0.8 mmol) (Dimethylamino)-2-(4-methylbenzoyl)-2-propennitril (das analog zu Beispiel 1 herstellbar ist), 0.68 mg (0.8 mmol) Piperidin und 20 ml 2-Pentanol werden 12 h lang bei 110 °C gerührt. Nach Abkühlen wird das Lösungsmittel im Vakuum verdampft und der Rückstand auf Kieselgel chromatographiert. Man erhält 217 mg (69.5 % d. Th.) der Titelverbindung. Smp.:229 °C

### Beispiel 15

### 3-(4,6-Diamino-5-benzylpyrimidin-2-yl)-1-(2-fluorbenzyl)-1H-pyrazolo [3,4-b]pyridin

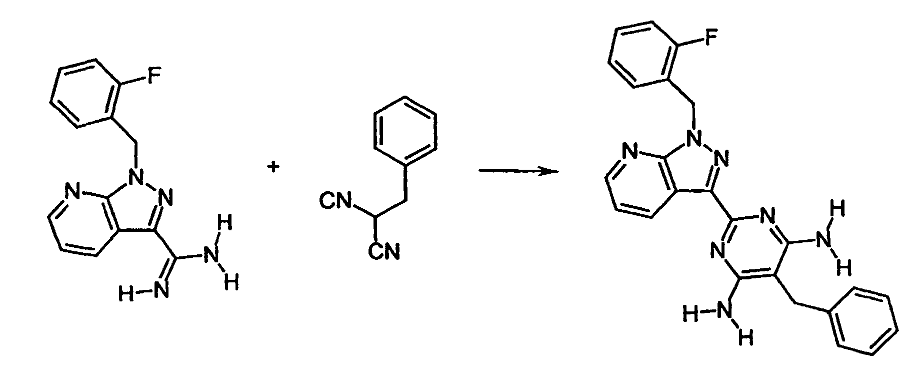

200 mg (0.74 mmol) 1-(2-Fluorbenzyl)-1H-pyrazolo[3,4-b]pyridin-3-carboxamidin aus Beispiel 25A, 125 mg (0.8 mmol) 2-Benzylmalodinitril (herstellbar aus Malodinitril und Benzylbromid mit einer Base wie Kaliumcarbonat), 0.68 mg (0.8 mmol) Piperidin und 20 ml 2-Pentanol werden 12 h lang bei 110 °C gerührt. Nach Abkühlen wird das Lösungsmittel im Vakuum verdampft und der Rückstand auf Kieselgel chromatographiert. Man erhält 165 mg (52.2 % d. Th.) der Titelverbindung.
Smp.:193 °C

### Beispiel 16

### 3-(4,6-Diamino-5-N-morpholinopyrimidin-2-yl)-1-(2-fluorbenzyl)-1H-pyrazolo[3,4-b]-pyridin

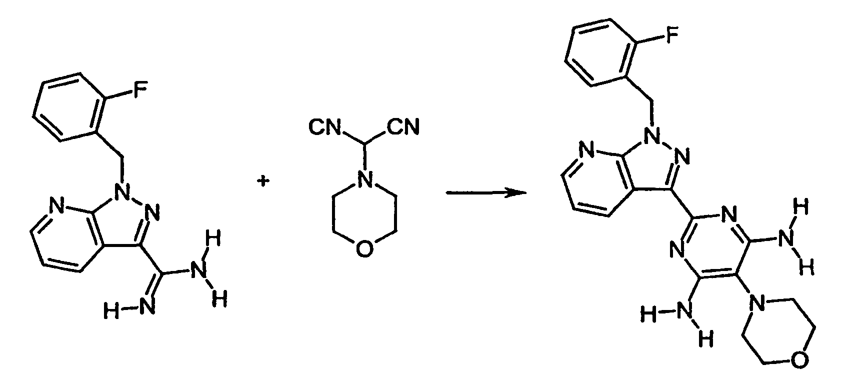

200 mg (0.74 mmol) 1-(2-Fluorbenzyl)-1H-pyrazolo[3,4-b]pyridin-3-carboxamidin aus Beispiel 25A und 400 mg (2.65 mmol) 2-N-Morpholinomalondinitril (Synthese vgl. H. Gold und O. Bayer, Chem. Ber. 1961, 94, 2594) werden bei 105°C 12 h im Vakuum erhitzt. Man löst den festen Rückstand in DMF, gibt Kieselgel hinzu und verdampft das Lösungsmittel im Vakuum. Nach Chromatographie erhält man 222 mg (71.1 % d.Th.) der Titelverbindung .
Smp: 261°C
Rf: (EE): 0.2

Analog wurden dargestellt:

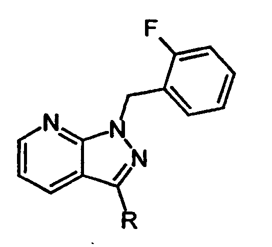

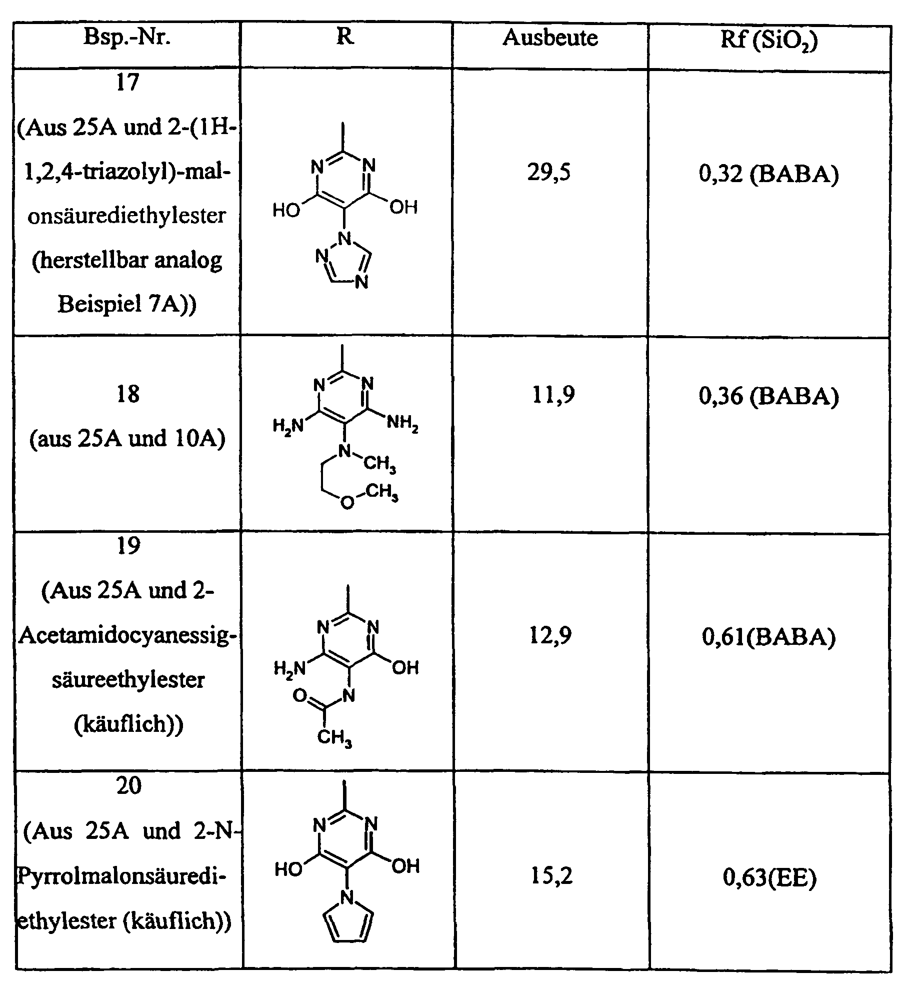

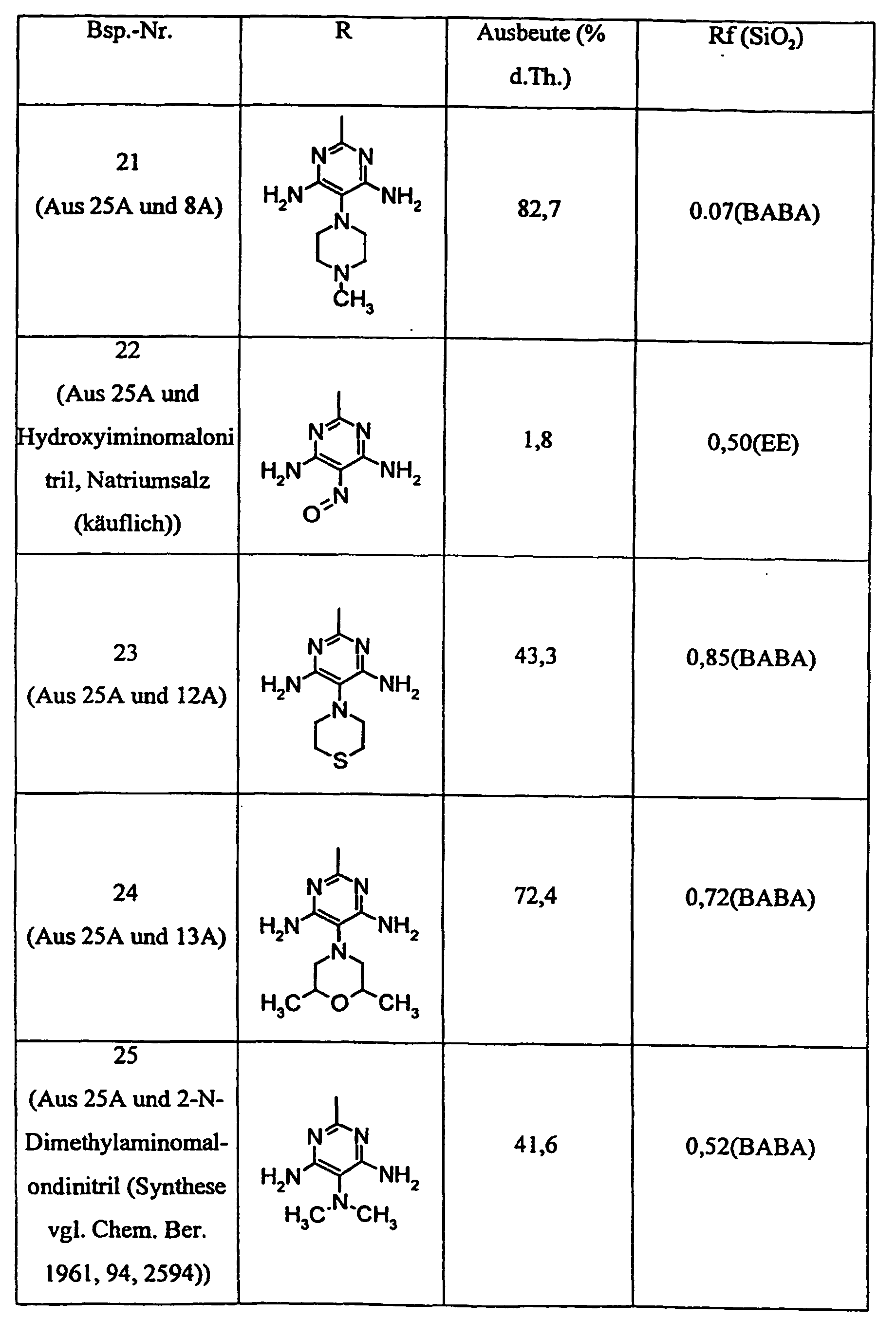

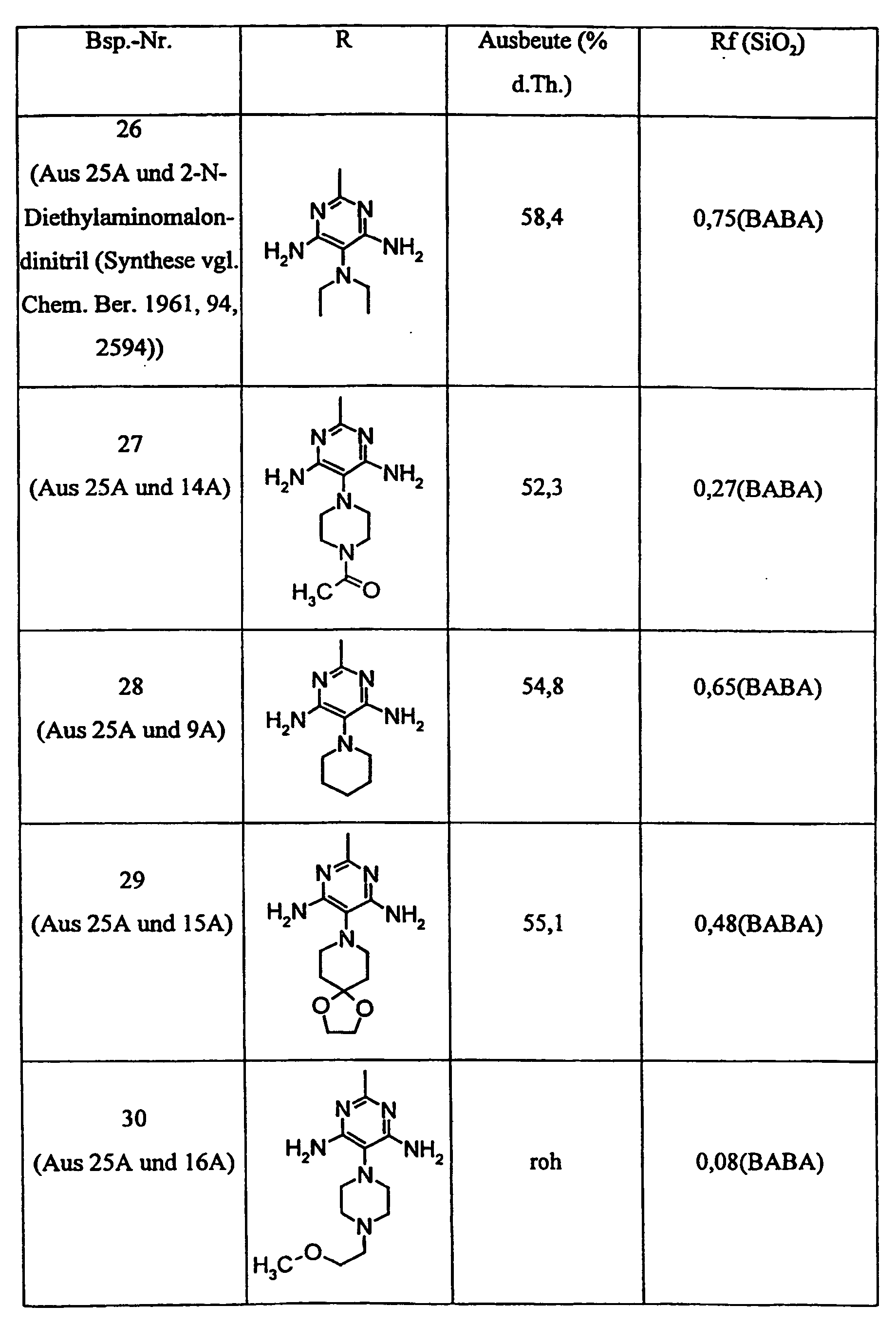

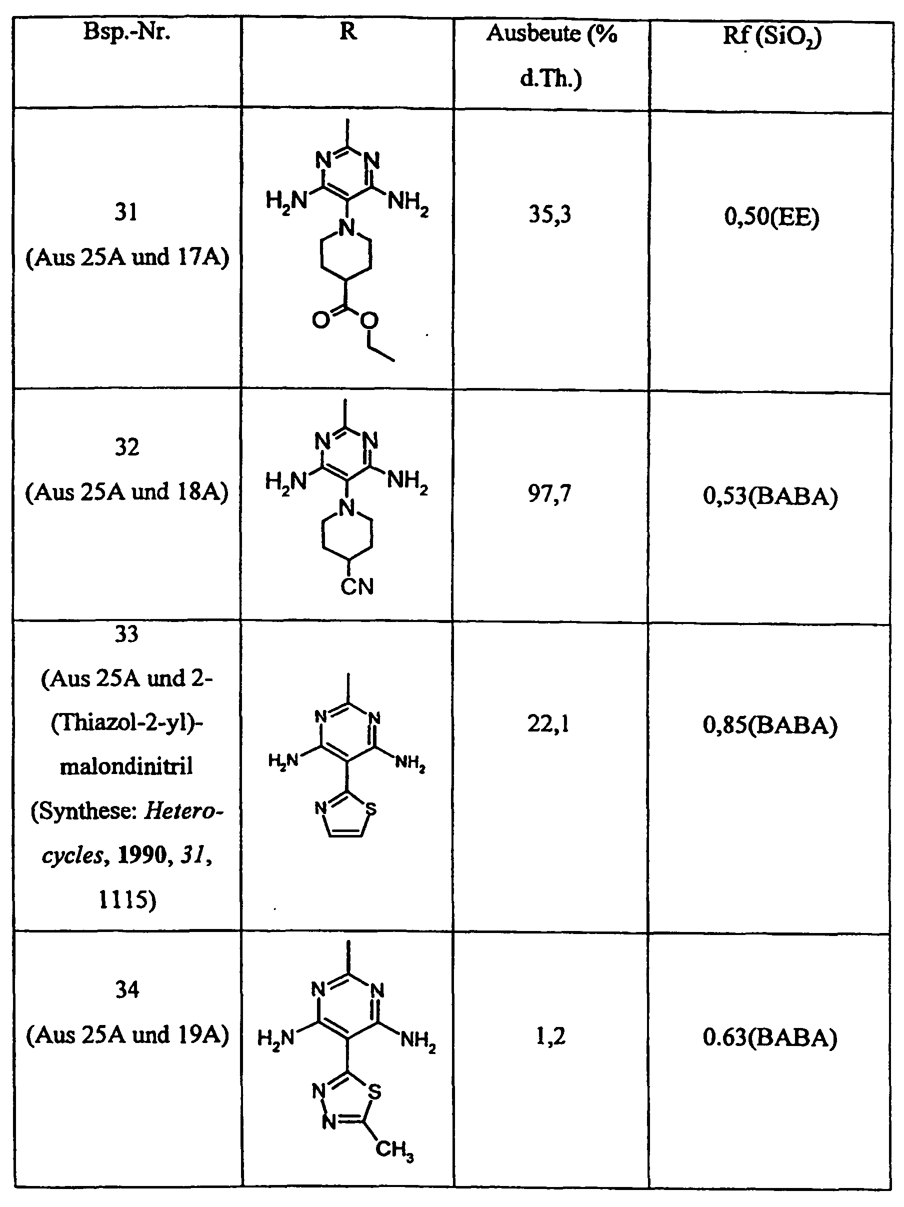

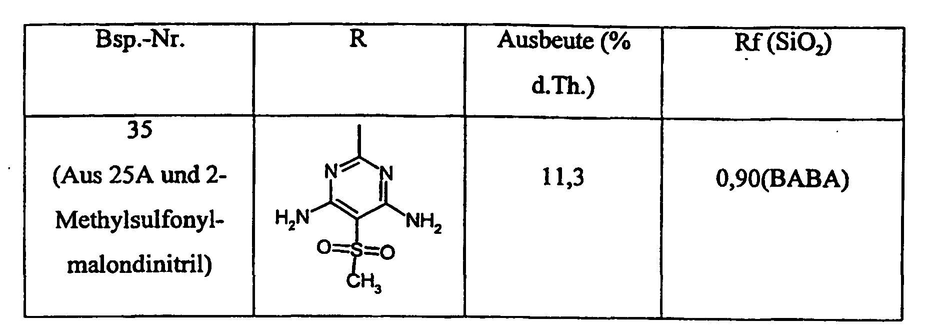

### Beispiel 36

### 3-(4,6-Bis(trifluormethyl)-pyrimidin-2-yl)-1-(2-fluorbenzyl)-1H-pyrazolo[3,4-b]pyridin

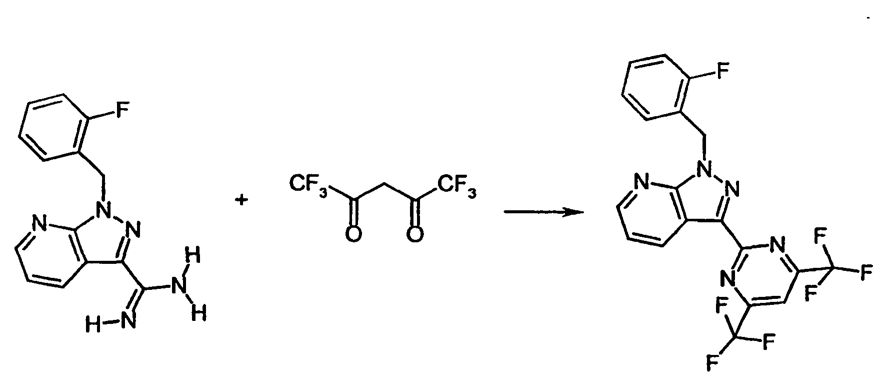

50 mg (19 mmol) 1-(2-Fluorbenzyl)-1H-pyrazolo[3,4-b]pyridin-3-carboxamidin aus Beispiel 25A und 42 mg (20 mmol) 1,1,1,5,5,5-Hexafluoracetylaceton werden bei 110°C 5 h erhitzt. Nach Chromatographie erhält man 33 mg (40.3 % d.Th.) der Titelverbindung .
Smp: 109°C
Rf: (Tol): 0.35

### Beispiel 37

### 3-(5-Ethoxycarbonyl-4-trifluormethyl-pyrimidin-2-yl)-1-(2-fluorbenzyl)-1H-pyrazolo[3,4-b]pyridin

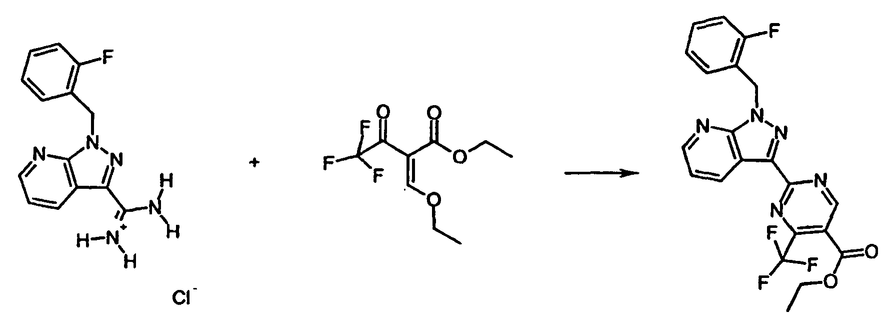

600 mg des rohen 1-(2-Fluorbenzyl)-1H-pyrazolo[3,4-b]pyridin-3-carboxamidin Hydrochlorids (herstellbar aus dem Amidin 25A durch Umsetzung mit HCl) werden in 30 ml Methanol mit 106 mg Natriummethanolat gerührt und mit 472 mg (1.96 mmol) 3-Ethoxy-2-trifluoroacetyl-acrylsäureethylester versetzt. Nach 12-stündigem Kochen wird der ausgefallene Niederschlag abgesaugt und mit Ether gewaschen. Man erhält 249 mg (27.5 % d. Th.) Kristalle.
Smp.: 174°C
Rf: SiO₂ T1E1: 0.76

### Beispiel 38

### 4-Amino-2-{1-(2-fluorbenzyl)-1H-pyrazolo[3,4-b]pyridin-3-yl}-pyrimidin-5-phosphonsäurediethylester

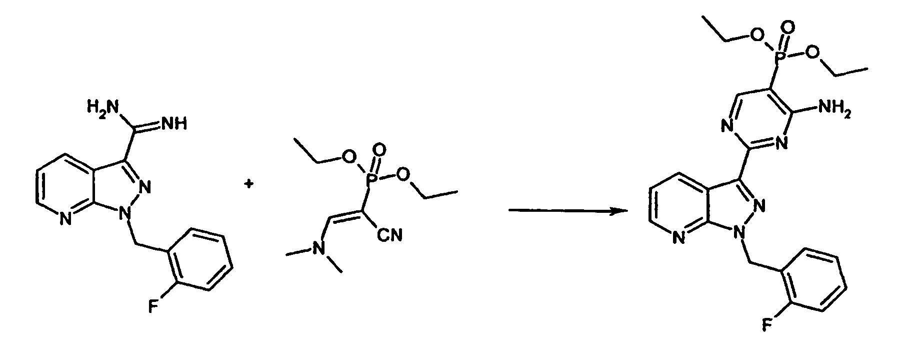

2,44 g (9.00 mmol) des Amidins aus Beispiel 25A und 3.71 g (16.0 mmol) 1-Cyano-1-(dimethylamino)methylen-methanphosphonsäurediethylester (Aboujaoude, Elie Elia; Collignon, Noel; Savignac, Philippe, Tetrahedron, 41, 1985, 427-434) wurden gut vermengt, 5 Minuten mit Ultraschall beschallt und anschließend über Nacht unter Vakuum (Membranpumpe) bei 100°C gerührt. Die auf Raumtemperatur abgekühlte Mischung wurde mit Methanol in der Hitze gerührt und von unlöslichen Bestandteilen durch Filtration befreit. Das Filtrat wurde eingeengt und an Kieselgel chromatographiert (C→ C:E 1:1 → E).
- Ausbeute:: 638 mg (16%)
- Smp.:: 185°C
- R_{f}-Wert:: 0.09 (C:E 1:1)

### Beispiel 39

### 3-(4-Amino-5-N-morpholino-pyrimidin-2-yl)-1-(2-fluorbenzyl)-1H-pyrazolo[3,4-b]pyridin

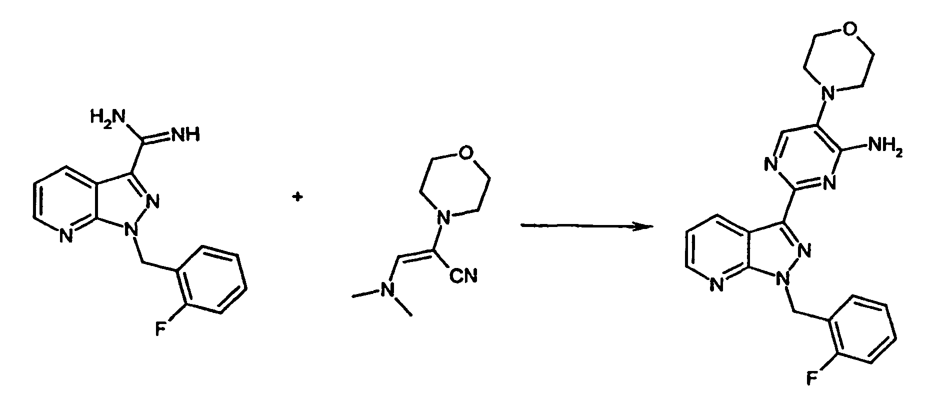

1.00 g (3.72 mmol) des Amidins aus Beispiel 25A und 2.00 g (11.0 mmol) 3-(Dimethylamino)-2-morpholino-acrylnitril aus Beispiel 3A wurden gut vermengt, 5 Minuten mit Ultraschall beschallt und anschließend über Nacht unter Vakuum (Membranpumpe) bei 120°C gerührt. Die auf Raumtemperatur abgekühlte Mischung wurde mit tert-Butyl-methylether verrührt, der entstandene Niederschlag abgesaugt und an Kieselgel chromatographiert (C:E 100:1 → C:E 1:1).
- Ausbeute:: 262 mg (17%)
- Smp.:: 205°C
- R_{f}-Wert:: 0.05 (C:E 1:1)

### Beispiel 40

### 3-(4-Amino-5-N-thiomorpholino-pyrimidin-2-yl)-1-(2-fluorbenzyl)-1H-pyrazolo [3,4-b]pyridin

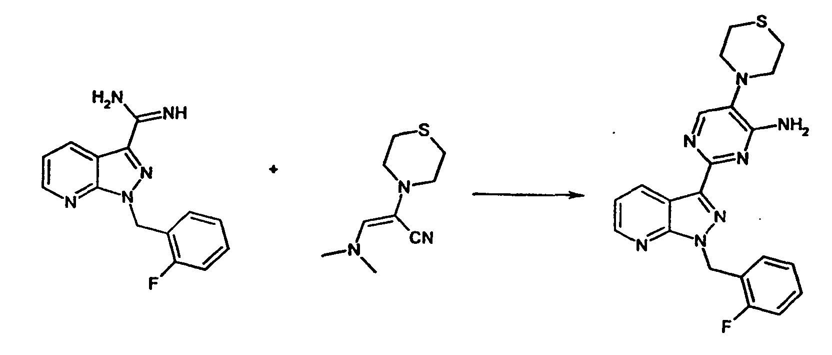

5.00 g (18.6 mmol) des Amidins aus Beispiel 25A und 9.98 g (50.7 mmol) 3-(Dimethylamino)-2-thiomorpholinoacrylnitril aus Beispiel 4A wurden gut vermengt, 5 Minuten mit Ultraschall beschallt und anschließend über Nacht unter Vakuum (Membranpumpe) bei 100°C gerührt. Die auf Raumtemperatur abgekühlte Mischung wurde mit tert-Butyl-methylether verrührt, der entstandene Niederschlag abgesaugt.
- Ausbeute:: 1.43 g (18%)
- Smp.:: >250°C
- R_{f}-Wert:: 0.06 (C:EE 1:1)

### Beispiel 41

### 3-(4-Hydroxy-5-(methylsulfonyl)-pyrimidin-2-yl)-1-(2-fluorbenzyl)-1H-pyrazolo[3,4-b]pyridin

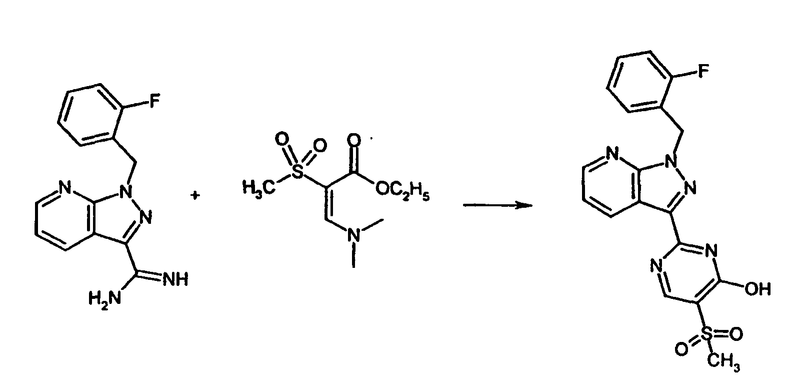

2.32 g (8.61 mmol) des Amidins aus Beispiel 25A und 5.71 g (25.8 mmol) des Enamins aus Beispiel 5A werden zusammengegeben und im offenen Gefäß bei 100-105°C 4 Stunden erhitzt. Der Rückstand wird abgekühlt, mit Toluol digeriert, filtriert und mit Toluol nachgewaschen.
Ausbeute: 1.16 g (33.6 % d. Th.).
Rf (SiO₂, EE): 0.23

### Beispiel 42

### 3-(6-Chloro-8-methyl-9H-purin-2-yl)-1-(2-fluorbenzyl)-1H-pyrazolo[3,4-b]pyridin

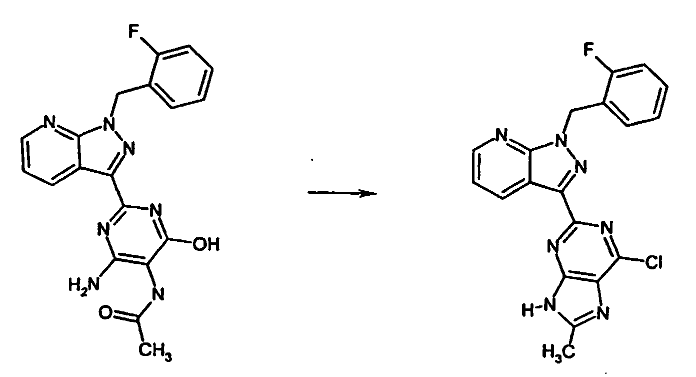

650 mg (1.65 mmol) des Hydroxypyrimidins aus Beispiel 19 werden in 3 mL Phosphoroxytrichlorid aufgenommen, zwei Tropfen N,N-Dimethylanilin werden zugegeben und die Lösung wird drei Stunden zum Rückfluß erhitzt. Die Reaktionsgemisch wird abgekühlt und einrotiert. Der Rückstand wird in Essigsäureethylester aufgenommen, vorsichtig mit gesättigter Natriumbicarbonatlösung gewaschen, getrocknet und einrotiert. Das Produkt wird roh weiter umgesetzt.
Ausbeute: 580 mg (89.1 % d. Th.).
Rf (SiO₂, EE): 0.21

Analog der Herstellung von Beispiel 42 wurden folgende Verbindungen dargestellt:

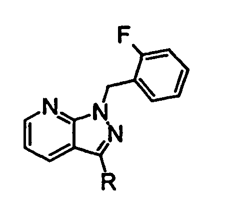

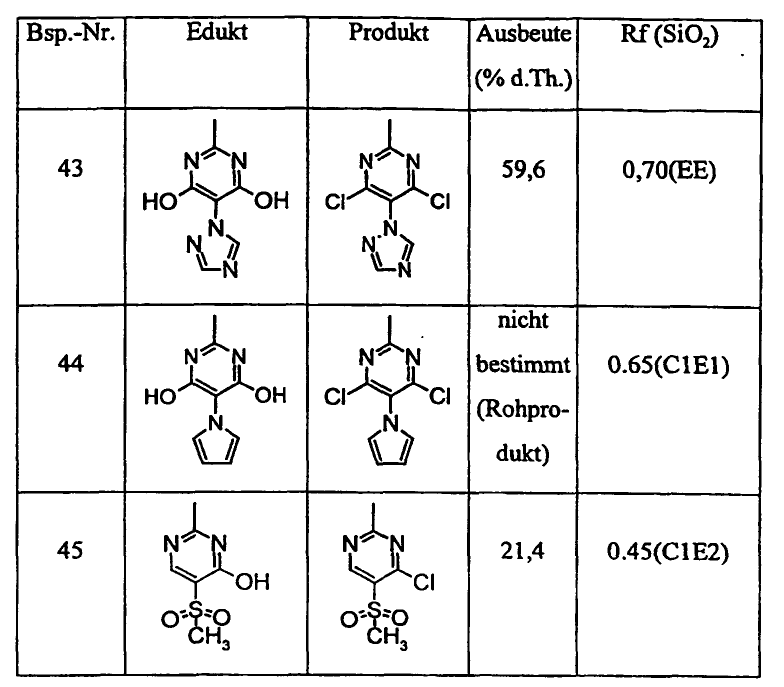

Das Edukt von Beispiel 43 wurde als Beispiel 17 hergestellt. Das Edukt von Beispiel 44 wurde als Beispiel 20 hergestellt. Das Edukt von Beispiel 45 wurde als Beispiel 41 hergestellt.

### Beispiel 46

### 3-(5-Ethyl-4-(2-hydroxyethylaminocarbonyl)pyrimidin- 2-yl)-1-(2-fluorbenzyl)-1H-pyrazolo[3,4-b]pyridin

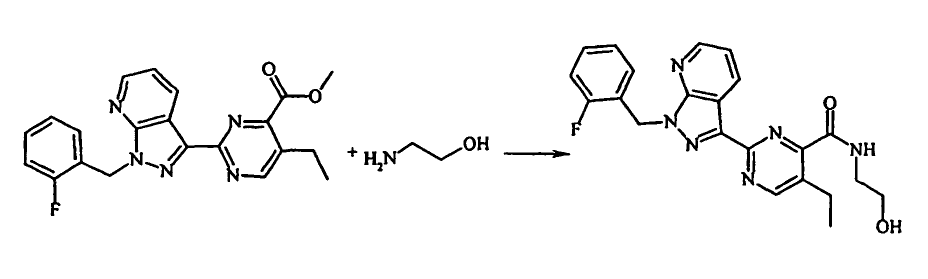

Man löst 70.0 mg (0.179 mmol) des Methylesters (hergestellt aus 25A und 4-(Dimethylamino)-3-ethyl-2-oxo-3-butensäuremethylester analog Beispiel 36) in 109.3 mg (1.78 mmol) des Amins und rührt 3 Stunden bei 60°C. Man gibt Dichloromethan hinzu und wäscht einmal mit 0.5N Salzsäurelösung. Die organische Phase wird mit Magnesiumsulfat getrocknet und im Vakuum verdampft. Ausbeute:
30.6 mg (40.7 % d. Th).
Rf(SiO₂, E) 0.31

Auf analoge Weise wurden durch Umsetzung mit den entsprechenden Aminen hergestellt:

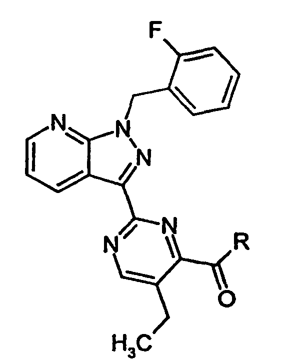

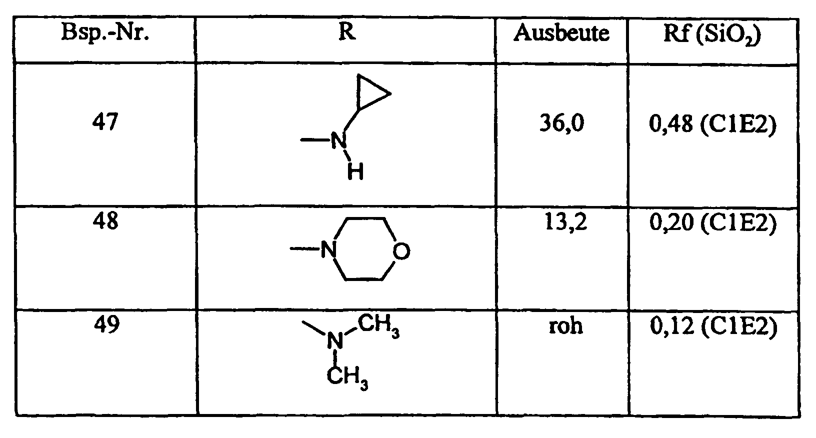

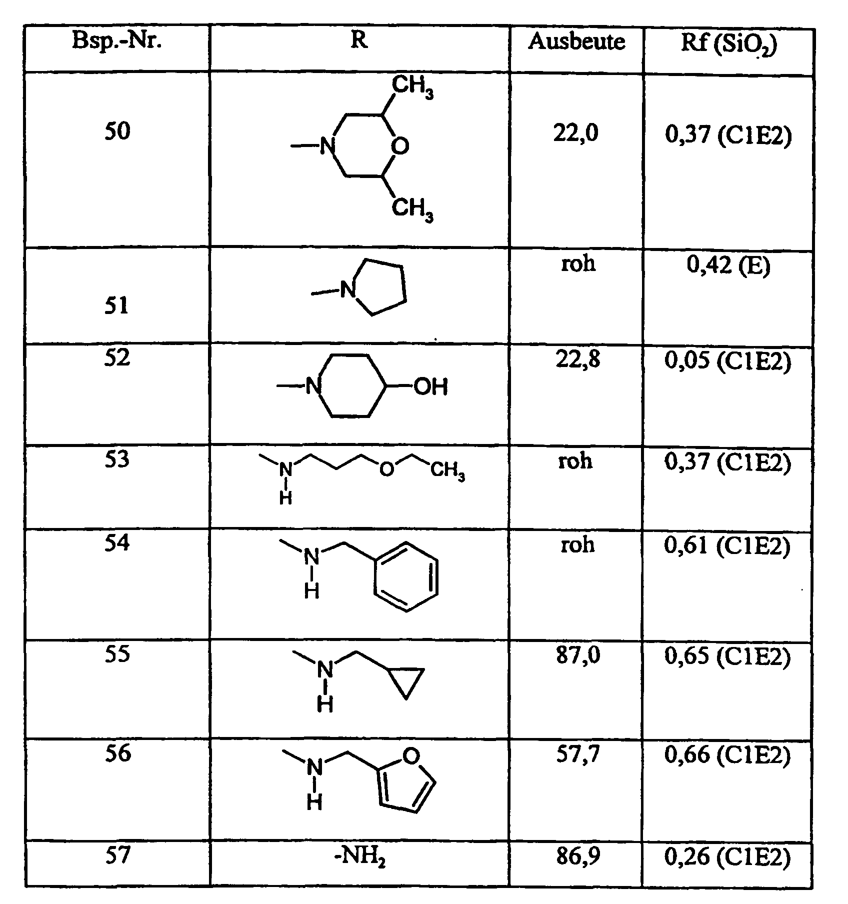

Die als Ausgangsverbindungen einzusetzenden Amine sind jeweils käuflich erhältlich oder auf dem Fachmann durch Standardverfahren, wie sie beispielsweise in J. March, Advanced Organic Chemistry, 3^{rd} ed. , Wiley, 1985, S. 1153 f. beschrieben sind, auf einfache Weise zugänglich.

### Beispiel 58

### 3-(4-(4,5-Dihydro-1H-imidazol-2-yl)-5-ethyl-pyrimidin-2-yl)-1-(2-fluorbenzyl)-1H-pyrazolo[3,4-b]pyridin

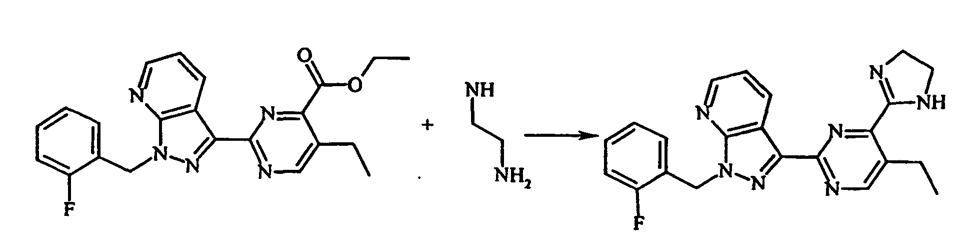

Man löst 68.9 mg (1.15 mmol) Ethylendiamin in 10 mL Toluol, gibt bei 0°C 0.60 mL (1.15 mmol) 2M Trimethylaluminiumlösung in Toluol zu und rührt 2 Stunden bei Raumtemperatur. Dann gibt man 155 mg (0.38 mmol) des Ethylesters (hergestellt aus 25A und 4-(Dimethylamino)-3-ethyl-2-oxo-3-butensäureethylester analog Beispiel 36) zu. Man rührt fünf Tage bei 75°C, kühlt ab, wäscht einmal mit Natriumkaliumtartrat-Lösung und extrahiert die wäßrige Phase einmal mit Dichlormethan. Die vereinigten organischen Phasen werden mit Magnesiumsulfat getrocknet, mit 500mg Kieselgel versetzt und einrotiert.

Die Substanz wird zur Reinigung an 10g Kieselgel 60 (Korngröße 0,040-0,063 mm) mit Essigester bis Essigester/Methanol 9:1 als Eluenten chromatographiert. Ausbeute 75.0 mg (49 % d. Th.).
Rf(SiO₂, C1E1): 0.04

### Beispiel 59

### 3-[5-ethyl- 4-(1H-imidazol-2-yl)-pyrimidin-2-yl]-1-(2-fluorbenzyl)-1H-pyrazolo[3,4-b]pyridin

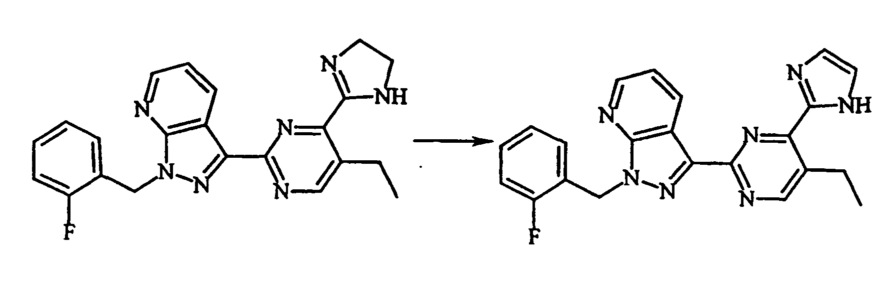

62 mg (0.15 mmol) des Dihydroimidazols aus Beispiel 58 und 100 mg Palladium/Kohle (10%ig) in 5 mL Toluol werden zum Rückfluß erhitzen. Nach 6 Tagen filtriert man ab und verdampft das Lösungsmittel im Vakuum.

Die Substanz wird zur Reinigung an 8g Kieselgel 60 (Korngröße 0,040-0,063 mm) mit Cyclohexan/Essigester 2:1 bis 1:2 als Eluenten chromatographiert. Ausbeute: 8.8 mg (14.3 % d. Th.)
Rf(SiO₂, C1E2): 0.24

### Beispiel 60

### 3-(5-Ethyl-4-(1H-imidazol-1-yl)-pyrimidin-2-yl)-1-(2-fluorbenzyl)-1H-pyrazolo[3,4-b]pyridin

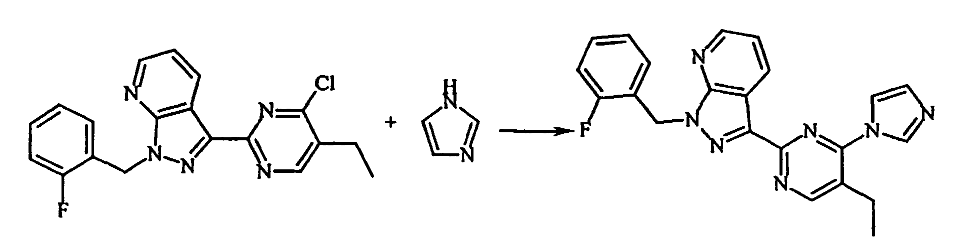

60 mg (0.16 mmol) der Chlorverbindung (hergestellt aus 25A und 2-Formylbutansäureethylester analog Beispiel 36 und anschließender Umsetzung mit Phosphoroxytrichlorid analog Beispiel 42) und 22.2 mg (0.33 mmol) Imidazol werden in 5 mL Dimethylformamid gelöst und mit 33.8 mg (0.24 mmol) Kaliumcarbonat versetzt. Man rührt über Nacht bei 100°C. Es wird abgekühlt, mit Essigester verdünnt und zweimal mit Wasser gewaschen. Die organische Phase wird mit Magnesiumsulfat getrocknet und im Vakuum eingedampft. Ausbeute: 47.4 mg (72.7 % d. Th).

Analog zu Beispiel 60 wurden durch Umsetzung mit den entsprechenden Aminen hergestellt:

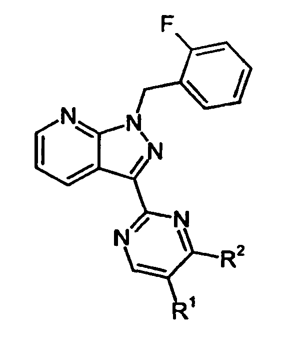

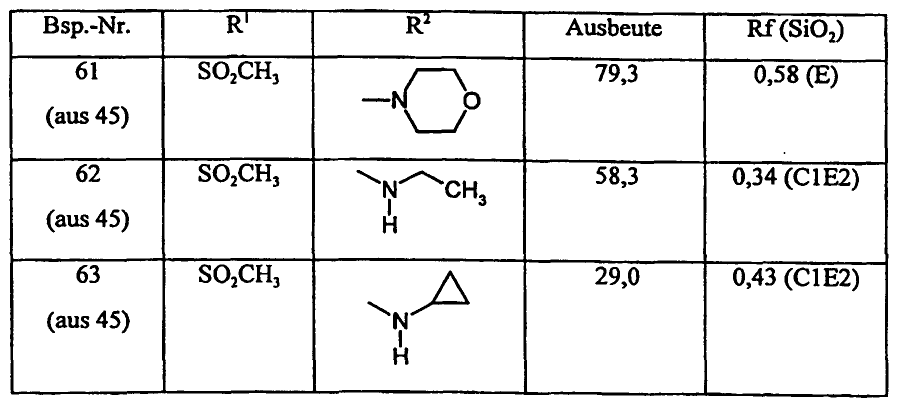

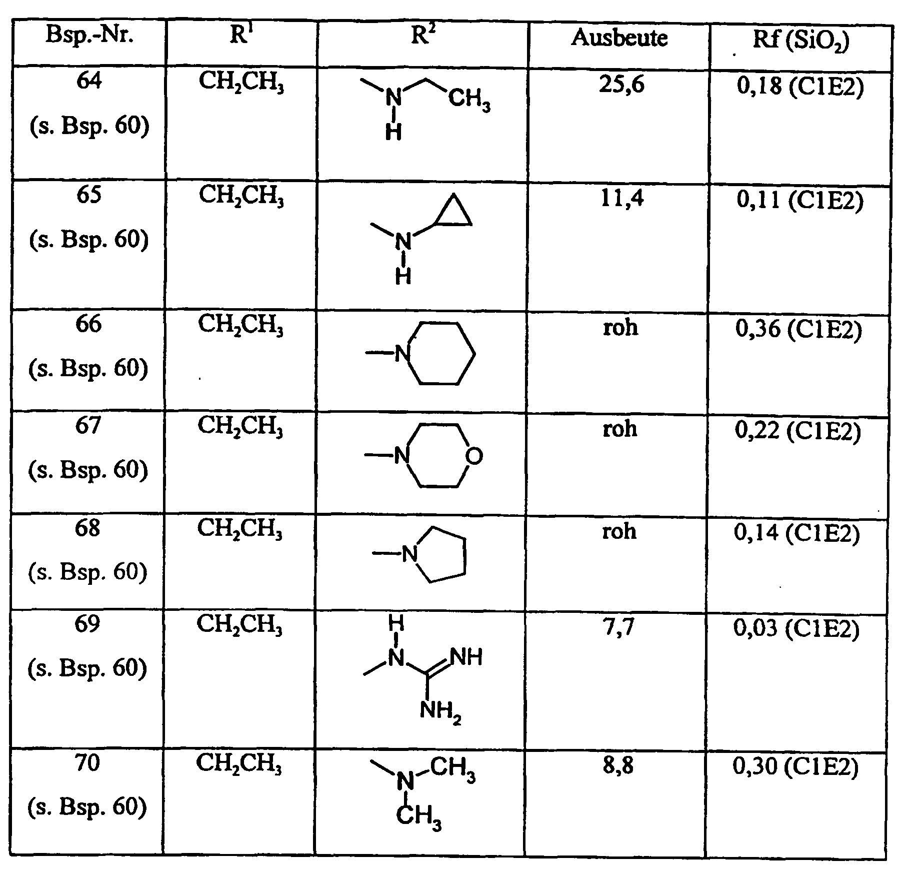

### Beispiele 71-79

Die Chlorgruppe der Verbindungen der Beispiele 42 bis 45 läßt sich nach bekannten Methoden mit Ammoniumformiat und Palladium/Kohle reduzieren oder durch Reaktion mit Nukleophilen wie dem Azidanion, Ammoniak, Aminen oder Methanol austauschen. Die so eingeführte Azidgruppe kann ihrerseits mit Natriumdithionit reduziert werden. Auf diese Weise erhält man folgende Verbindungen:

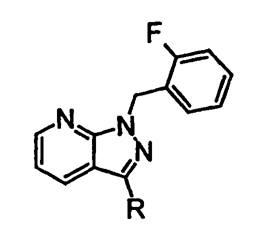

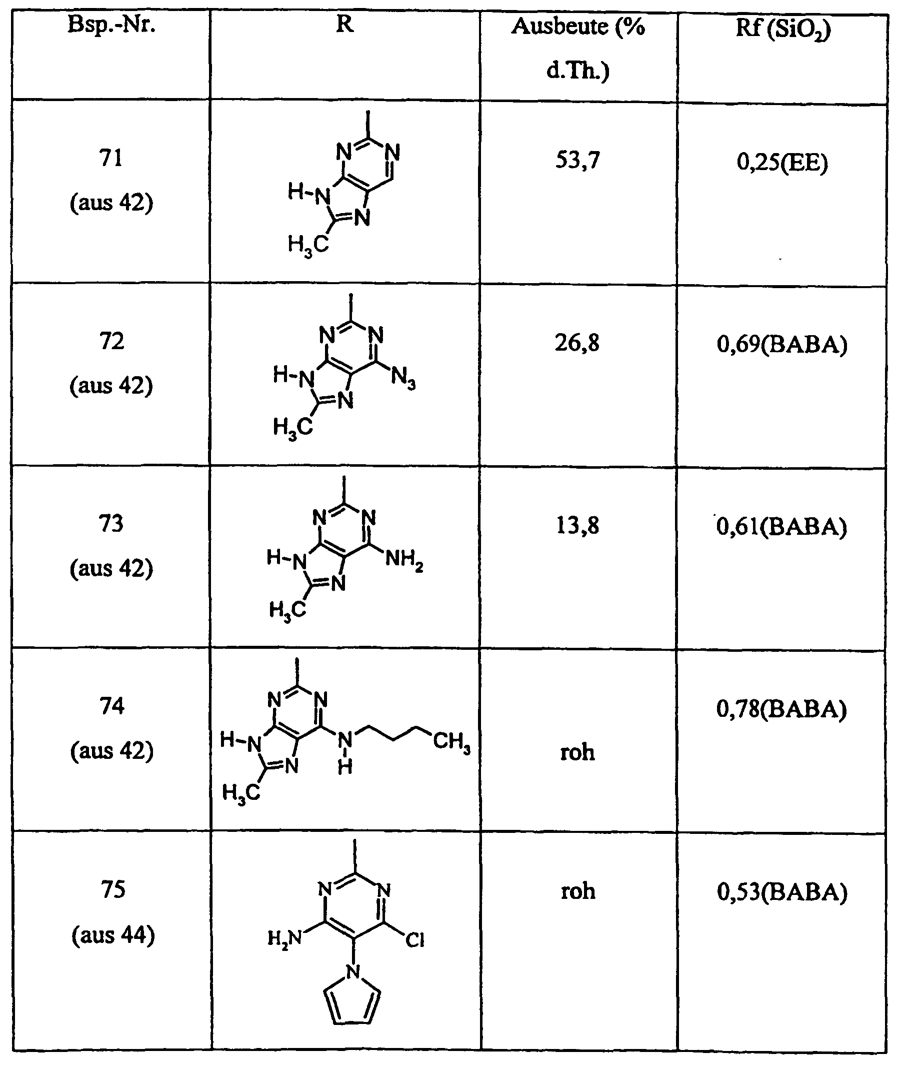

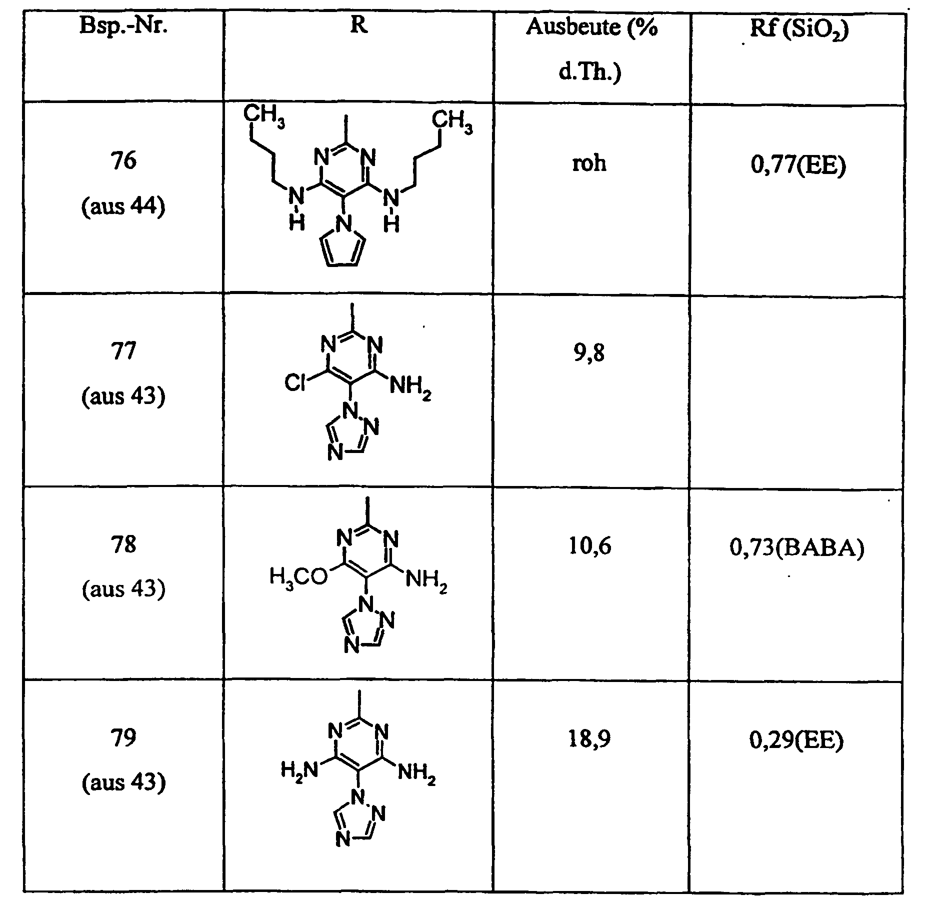

### Beispiel 80

### 3-(4-Diacetylamino-5-ethyl-pyrimidin-2-yl)-1-(2-fluorobenzyl)-1H-pyrazolo[3,4-b]pyridin

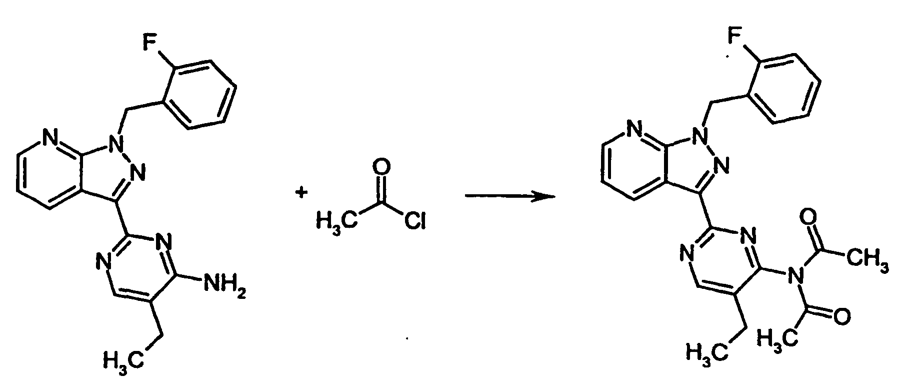

50.0 mg (0.14 mmol) des Amins (herstellbar analog zu Beispiel 60 durch Umsetzung der dort beschriebenen Chlorverbindng mit Ammoniak beziehungsweise mit Natriumazid und anschließender Reduktion mit Natriumdithionit) werden in Dichlormethan gelöst und mit 33.8 mg (0.43 mmol) Acetylchlorid und 68.1 mg (0.86 mmol) Pyridin versetzt. Die Lösung wird vier Stunden bei RT verrührt, einmal mit 1N HCl, dann mit gesättiger NaHCO₃-Lösung gewaschen. Die organische Phase wird mit Magnesiumsulfat getrocknet und im Vakuum eingedampft. Die Substanz wurde zur Reinigung an Kieselgel 60 (Korngröße 0,040-0,063 mm) mit Cyclohexan/Essigester 1:1 als Eluenten chromatographiert. Ausbeute: 33.2 mg (53.5% d. Th.)
Rf (SiO₂, C1E2): 0.41

### Beispiel 81

### 3-[4-(2-Benzoyloxymethylbenzoylamino)-5-ethylpyrimidin-2-yl]-1-(2-fluorbenzyl-1H-pyrazolo[3,4-b]pyridin

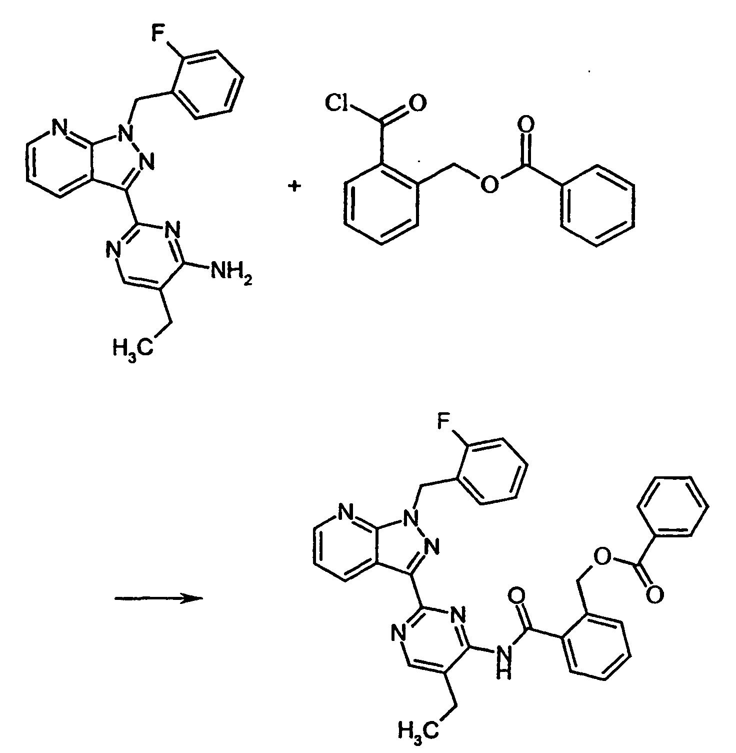

9 mg (0,23 mmol) Natriumhydrid (60%-ige ölige Suspension) werden bei Raumtemperatur in 1 ml Tetrahydrofuran (THF) suspensiert. Eine Lösung von 40 mg (0,11 mmol) des Amins (vgl. Beispiel 80) in 0,8 ml THF wird zugegeben, und anschließend wird eine Lösung von 34,7 mg (0,13 mmol) 2-(Benzoyloxymethyl)benzoylchlorid zugegeben. Nach 30 min werden 5 ml (0,12 mmol) Natriumhydrid (60%-ig) und 14 mg (0,05 mmol) des vorstehenden Säurechlorids erneut zugegeben. Nach 1 h wird die Mischung mit Wasser versetzt, mit Essigsäureethylester extrahiert und die organische Phase mit 1M Salzsäure und gesättigter NaHCO₃-Lösung gewaschen, mit Magnesiumsulfat getrocknet und im Vakuum eingedampft. Die Substanz wird aus Cyclohexan/Essigsäureethylester umkristallisiert.
Ausbeute: 25 mg (37,1% d. Theorie)
Rf (SiO₂, C1E1): 0,50

### Beispiel 82

### 3-(4-Acetoxy-5-ethyl-pyrimidin-2-yl)-1-(2-fluorbenzyl)-1H-pyrazolo[3,4-b]pyridin und 3-(3-acetyl-5-ethyl-pyrimidin-4-on-2-yl)-1-(2-fluorbenzyl)-1H-pyrazolo[3,4-b]-pyridin

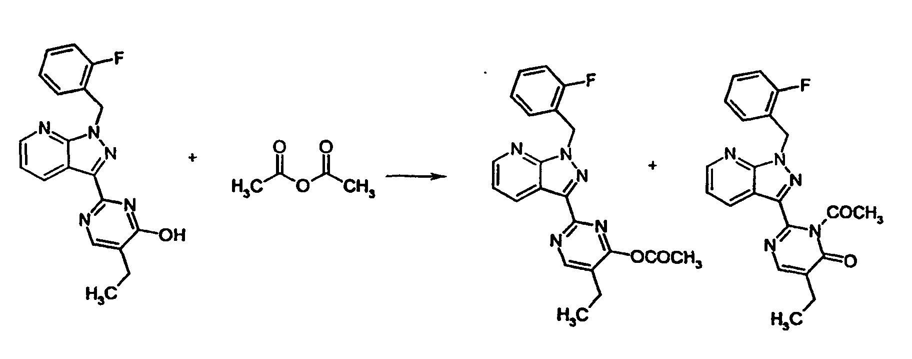

73.8 mg (0.21 mmol) der Hydroxypyrimidinverbindung (hergestellt aus 25A und 2-Formylbutansäureethylester analog Beispiel 36) werden in 2 mL Dichlormethan gelöst und mit 27.9 mg (0.27 mmol) Triethylamin und 25.9 mg (0.25 mmol) Essigsäureanhydrid versetzt. Die Lösung wird drei Stunden bei RT gerührt, in Essigester aufgenommen, einmal mit Wasser gewaschen und die wäßrige Phase einmal mit Essigester extrahiert. Die vereinigten organischen Phasen werden noch zweimal mit Wasser gewaschen, mit Magnesiumsulfat getrocknet und einrotiert. Ausbeute: 42.0 mg (50.8% d. Th.)
Rf (SiO₂, C1E2): 0.5

### Beispiel 83

### 3-(5-Ethyl-4-(methylsulfinyl)-pyrimidin-2-yl)-1-(2-fluorbenzyl)-1H-pyrazolo[3,4-b] pyridin und 3-(5-Ethyl-4-(methylsulfonyl)pyrimidin-2-yl)-1-(2-fluorbenzyl)-1H-pyrazolo[3,4-b]pyridin

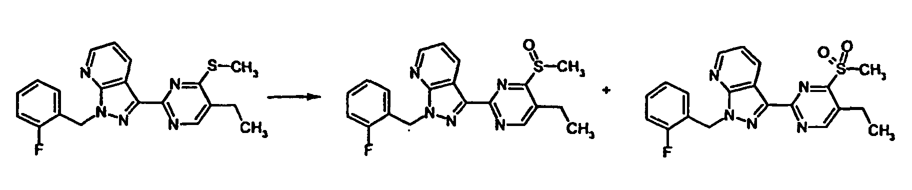

45.2 mg (0.12 mmol) des Methylthioethers (hergestellt aus der in Beispiel 60 verwendeten Chlorverbindung durch Umsetzung mit Natriummethanthiolat in Toluol) und 30.8 mg (0.18 mmol) MCPBA in 2 mL Dichlormethan werden bei 0°C gerührt. Nach drei Stunden wird das Reaktionsgemisch mit Natriumbicarbonatlösung und Essigester versetzt, abgetrennt, getrocknet und einrotiert.

Die Substanz wird zur Reinigung an 8 g Kieselgel 60 (Korngröße 0,040-0,063 mm) mit Cyclohexan/Essigester 1:1 bis 1:4 als Eluenten chromatographiert.
B: Ausbeute: 36.0 mg (76.4 % d. Th.). Rf (SiO₂, C1E2): 0.057
C: Ausbeute: 7.1 mg (14.5 % d. Th.). Rf (SiO₂, C1E2): 0.79

### Beispiel 84

### 3-(4,6-Diamino-5-N-4-oxothiomorpholinopyrimidin-2-yl)-1-(2-fluorbenzyl)-1H-pyrazolo[3,4-b]pyridin und 3-(4,6-Diamino-5 N-4,4-dioxothiomorpholinopyrimidin-2-yl)-1-(2-fluorbenzyl)-1H-pyrazolo[3,4-b]pyridin

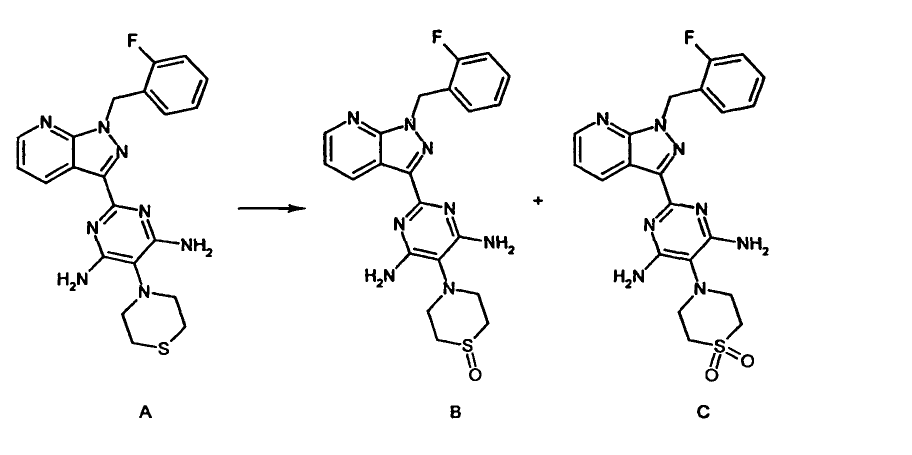

230 mg (0.53 mmol) des Thiomorpholins aus Beispiel 23 und 130 mg (0.53 mmol) MCPBA werden in 5 mL Dichlormethan bei 0°C gerührt. Nach 30 min. wird die gleiche Menge MCPBA erneut zugegeben. Nach 1.5 Stunden wird das Reaktionsgemisch mit Kieselgel versetzt und einrotiert. Die Substanz wird zur Reinigung an Kieselgel 60 (Korngröße 0,040-0,063 mm) mit Cyclohexan/Essigester chromatographiert.
B: Ausbeute: 86 mg (36.1 % d. Th.). Rf (SiO₂, BABA): 0.18
C: Ausbeute: 14 mg (5.7 % d. Th.). Rf(SiO₂, BABA): 0.41

## Patentansprüche

1. Substituierte Pyrazol-Derivate der allgemeinen Formel (I) 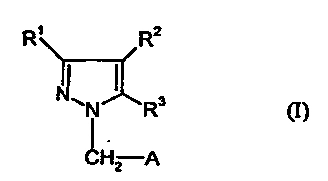 in welcher
R² und R³ unter Einbezug der Doppelbindung einen Pyridyl-oder Pyrimidinylring bilden,
A für Phenyl oder Pyrimidyl steht, die gegebenenfalls durch Fluor, Chlor oder Brom substituiert sind,
R¹ für einen Rest der Formel 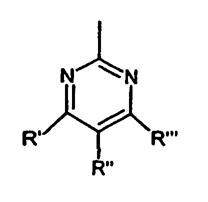 steht,
worin
R' für NH₂ steht,
R" für gegebenenfalls substituiertes Morpholino, Piperidin, Piperazin, Pyrrolidin, Triazolyl oder Thiomorpholino steht
und
R"' für Wasserstoff oder NH₂ steht

2. Verbindungen nach Anspruch 1, wobei R" für Morpholinyl steht.

3. Verfahren zur Herstellung der Verbindungen der allgemeinen Formel (I) nach Anspruch 1,
**dadurch gekennzeichnet, daß**
man in Abhängigkeit der verschiedenen Bedeutungen der oben unter R² und R³ aufgeführten Heterocyclen
[A] Verbindungen der allgemeinen Formel (II) (II)
R¹-D (II)
in welcher
R¹ die oben angegebene Bedeutung hat,
und
D für Reste der Formeln 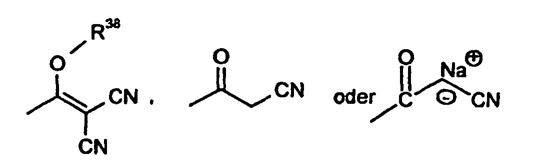
steht,
in welchen
R³⁸ für C₁-C₄-Alkyl steht,
durch Umsetzung mit Verbindungen der allgemeinen Formel (III)
A-CH₂-NH-NH₂ (III)
in welcher
A die oben angegebene Bedeutung hat
in inerten Lösemitteln, gegebenenfalls in Anwesenheit einer Base, in die Verbindungen der allgemeinen Formel (IV) oder (IVa) 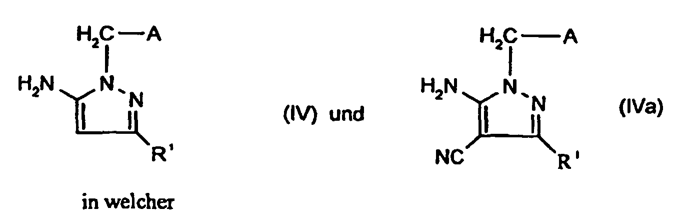
A und R¹ die oben angegebene Bedeutung haben,
überführt,
und im Fall der Verbindungen der allgemeinen Formel (IVa) anschließend mit Carbonsäuren, Nitrilen, Formamiden oder Guanidiniumsalzen cyclisiert,
und im Fall der Verbindungen der allgemeinen Formel (IV) mit 1,3-Dicarbonyl-Derivaten, deren Salze, Tautomeren, Enolether oder Enaminen, in Anwesenheit von Säuren und gegebenenfalls unter Mikrowellen cyclisiert,
oder
[C] Verbindungen der allgemeinen Formel (VII) 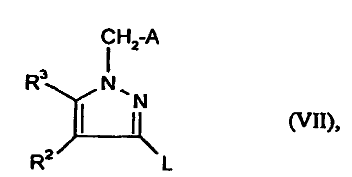 in welcher
A¹, R² und R³ die oben angegebene Bedeutung haben,
und
L für einen Rest der Formel -SnR³⁹R⁴⁰R⁴¹, ZnR⁴², lod, Brom oder Triflat steht,
worin
R³⁹, R⁴⁰ und R⁴¹ gleich oder verschieden sind und geradkettiges oder verzweigtes Alkyl mit bis zu 4 Kohlenstoffatomen bedeuten,
und
R⁴² Halogen bedeutet,
mit Verbindungen der allgemeinen Formel (VIII) (VIII),
R¹-T (VIII)
in welcher
R¹ die oben angegebene Bedeutung hat
und
im Fall L = SnR³⁹R⁴⁰R⁴¹ oder ZnR⁴²
T für Triflat oder für Halogen, vorzugsweise für Brom steht,
und
im Fall L = lod, Brom oder Triflat
T für einen Rest der Formel SnR^{39'}R^{40'}R^{41'}, ZnR^{42'} oder BR^{43'}R^{44'} steht,
worin
R³⁹, R^{40'}, R^{41'} und R^{42'} die oben angebene Bedeutung von R³⁹, R⁴⁰, R⁴¹ und R⁴² haben und mit diesen gleich oder verschieden sind,
R⁴³ und R^{44'} gleich oder verschieden sind und Hydroxy, Aryloxy mit 6 bis 10 Kohlenstoffatomen oder geradkettiges oder verzweigtes Alkyl oder Alkoxy mit jeweils bis zu 5 Kohlenstoffatomen bedeuten, oder gemeinsam einen 5- oder 6-gliedrigen carbocyclischen Ring bilden,
in einer palladiumkatalysierten Reaktion in inerten Lösemitteln umsetzt, gegebenenfalls in Gegenwart einer Base,
oder
[D] im Fall, daß R¹ für einen gegebenenfalls substituierten Pyrimidinrest steht, Amidine der allgemeinen Formel (IX) 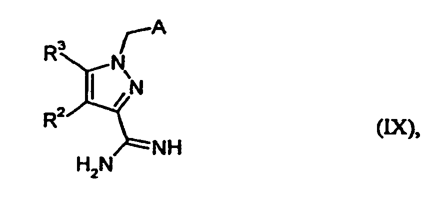 in welcher
A, R² und R³ die oben angegebene Bedeutung haben,
beispielsweise mit Verbindungen der allgemeinen Formel (X), (Xa), (Xb) oder (Xc) 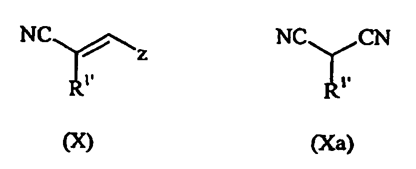 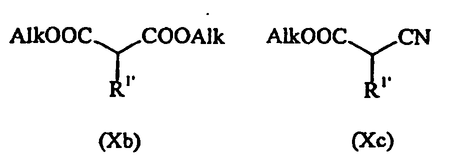 in welchen
R^{1'} für den oben unter R¹ aufgeführten gegebenenfalls substituierten Cycloalkylrest steht;
Alk für geradkettiges oder verzweigtes Alkyl mit bis zu 8 Kohlenstoffatomen, vorzugsweise bis zu vier Kohlenstoffatomen steht;
und
Z für eine NH₂-Gruppe, Monoalkylaminogruppe mit bis zu 7 Kohlenstoffatomen, Dialkylaminogruppe mit bis zu 7 Kohlenstoffatomen, einen über den Stickstoff gebundenen Piperidino- oder Morpholinorest, Hydroxyl, Alkoxy mit bis zu 7 Kohlenstoffatomen, Acyloxy mit bis zu 7 Kohlenstoffatomen oder Aroyloxy mit 6 bis 10 Kohlenstoffatomen steht,
umsetzt,
und gegebenenfalls die unter X, Y, R¹, R², R³ und/oder A aufgeführten Substituenten nach üblichen Methoden, vorzugsweise durch Acylierung freier Aminogruppen oder Hydroxygruppen, Chlorierung, katalytische Hydrierung, Reduktion, Oxidation, Abspaltung von Schutzgruppen und/oder nucleophiler Substitution variiert oder einführt.

4. Arzneimittel enthaltend mindestens eine Verbindung der allgemeinen Formel (I) gemäß Anspruch 1.

5. Verfahren zur Herstellung von Arzneimitteln **dadurch gekennzeichnet, daß** man mindestens eine Verbindung der Formel (I) gemäß Anspruch 1, gegebenenfalls mit üblichen Hilfs- und Zusatzstoffen in eine geeignete Applikationsform überführt.

6. Armeimittel enthaltend mindestens eine Verbindung der allgemeinen Formel (I) gemäß Anspruch 1 in Kombination mit organischen Nitraten oder NO-Donatoren.

7. Arzneimittel enthaltend mindestens eine Verbindung der allgemeinen Formel (I) gemäß Anspruch 1 in Kombination mit Verbindungen, die den Abbau von cyclischen Guanosinmonophosphat (cGMP) inhibieren.

8. Verwendung von Verbindungen der allgemeinen Formel (I) gemäß Anspruch 1 zur Herstellung von Arzneimitteln.

9. Verwendung von Verbindungen der allgemeinen Formel (I) gemäß Anspruch 1 bei der Herstellung von Arzneimitteln zur Behandlung von Herz-Kreislauf-Erkrankungen.

10. Verwendung von Verbindungen der allgemeinen Formel (I) gemäß Anspruch 1 bei der Herstellung von Arzneimitteln zur Behandlung von Hypertonie.

11. Verwendung von Verbindungen der allgemeinen Formel (I) gemäß Anspruch 1 bei der Herstellung von Arzneimitteln zur Behandlung von thromboembolischen Erkrankungen und Ischämien.

12. Verwendung von Verbindungen der allgemeinen Formel (I) gemäß Anspruch 1 bei der Herstellung von Arzneimitteln zur Behandlung von sexueller Dysfunktion.

13. Verwendung von Verbindungen der allgemeinen Formel (I) gemäß Anspruch 1 bei der Herstellung von Arzneimitteln mit anitinflammatorischen Eigenschaften.

14. Verwendung gemäß einem der Ansprüche 8 bis 13, wobei die Verbindungen der allgemeinen Formel (I) gemäß Anspruch 1 in Kombination mit organischen Nitraten oder NO-Donatoren oder in Kombination mit Verbindungen, die den Abbau von cyclischen Guanosinmonophosphat (cGMP) inhibieren, eingesetzt werden.

## Claims

1. Substituted pyrazole derivatives of the general formula (I) 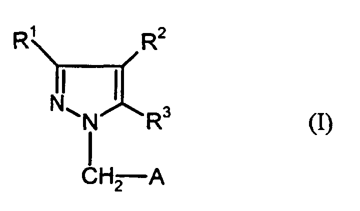 in which
R² and R³ together with the double bond form a pyridyl or pyrimidinyl ring,
A represents phenyl or pyrimidyl, which are optionally substituted by fluorine, chlorine or bromine,
R¹ represents a radical of the formula 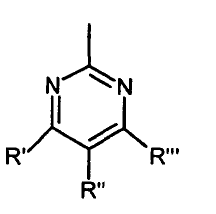 in\which
R' represents NH₂,
R" represents optionally substituted morpholinyl, piperidinyl, piperazinyl, pyrrolidinyl, triazolyl or thiomorpholinyl
and
R''' represents hydrogen or NH₂.

2. Compounds according to Claim 1 in which R" represents morpholinyl.

3. Process for preparing the compounds of the general formula (I) according to Claim 1,
**characterized in that**
depending on the various meanings of the heterocycles listed above under R² and R³
[A] compounds of the general formula (II)
R¹-D (II)
in which
R¹ is as defined above
and
D represents radicals of the formulae 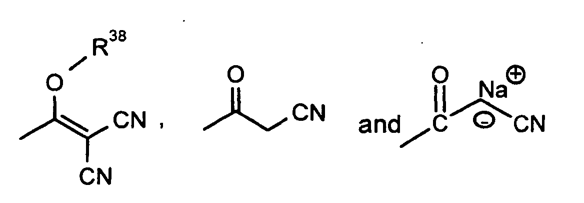
in which
R³⁸ represents C₁-C₄-alkyl
are converted, by reaction with compounds of the general formula (III)
A-CH₂-NH-NH₂ (III)
in which
A is as defined above
in inert solvents, if appropriate in the presence of a base, into the compounds of the general formula (IV) or (IVa) 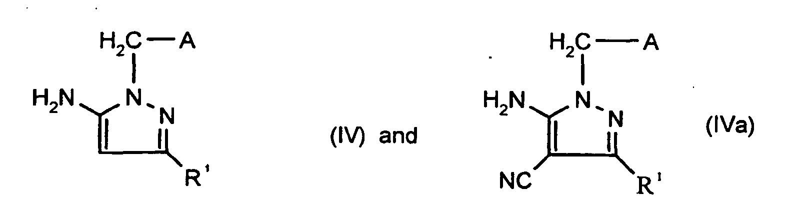 in which
A and R¹ are each as defined above,
and, in the case of the compounds of the general formula (IVa), are subsequently cyclized with carboxylic acids, nitriles, formamides or guanidium salts,
and, in the case of the compounds of the general formula (IV), are cyclized with 1,3-dicarbonyl derivatives, their salts, tautomers, enol ethers or enamines, in the presence of acids and, if appropriate, under microwave irradiation,
or
[C] compounds of the general formula (VII) 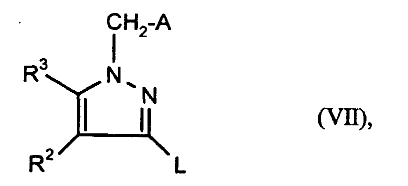 in which
A¹, R² and R³ are each as defined above
and
L represents a radical of the formula -SnR³⁹R⁴⁰R⁴¹, ZnR⁴², iodine, bromine or triflate
in which
R³⁹, R⁴⁰ and R⁴¹ are identical or different and each represents straight-chain or branched alkyl having up to 4 carbon atoms
and
R⁴² represents halogen
are reacted with compounds of the general formula (VIII)
R¹-T (VIII),
in which
R¹ is as defined above
and
in the case that L = SnR³⁹R⁴⁰R⁴¹ or ZnR⁴²,
T represents triflate or represents halogen, preferably bromine
and,
in the case that L = iodine, bromine or triflate,
T represents a radical of the formula SnR^{39'}R^{40'}R^{41'}, ZnR^{42'} or BR^{43'}R^{44'}
in which
R^{39'}, R⁴⁰', R⁴¹' and R^{42'} have the meanings of R³⁹, R⁴⁰, R⁴¹ and R⁴² given above and are identical to or different from them,
R^{43'} and R^{44'} are identical or different and each represents hydroxyl, aryloxy having 6 to 10 carbon atoms or straight-chain or branched alkyl or alkoxy having in each case up to 5 carbon atoms, or together form a 5- or 6-membered carbocyclic ring
in a palladium-catalysed reaction in inert solvents, if appropriate in the presence of a base,
or
[D] in the case that R¹ represents an optionally substituted pyrimidine radical, amidines of the general formula (IX) 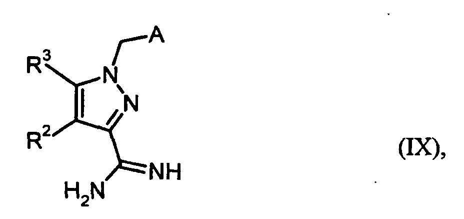 in which
A, R² and R³ are each as defined above
are reacted, for example, with compounds of the general formula (X), (Xa), (Xb) or (Xc) 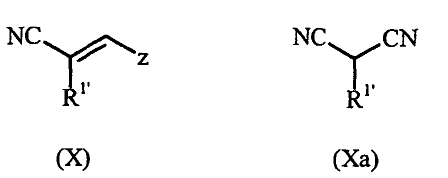 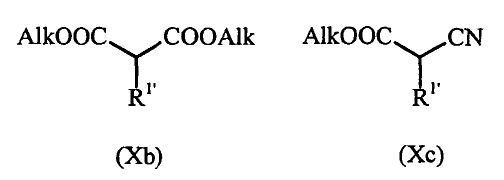 in which
R^{1'} represents the optionally substituted cycloalkyl radical listed above under R¹;
Alk represents straight-chain or branched alkyl having up to 8 carbon atoms, preferably up to 4 carbon atoms;
and
Z represents an NH₂ group, a monoalkylamino group having up to 7 carbon atoms, a dialkylamino group having up to 7 carbon atoms, a piperidinyl or morpholinyl radical which is attached via the nitrogen, hydroxyl, alkoxy having up to 7 carbon atoms, acyloxy having up to 7 carbon atoms or aroyloxy having 6 to 10 carbon atoms,
and, if appropriate, the substituents listed under X, Y, R¹, R², R³ and/or A are modified or introduced by customary methods, preferably by acylation of free amino groups or hydroxyl groups, chlorination, catalytic hydrogenation, reduction, oxidation, removal of protective groups and/or nucleophilic substitution.

4. Medicaments, comprising at least one compound of the general formula (I) according to Claim 1.

5. Process for preparing medicaments, **characterized in that** at least one compound of the formula (I) according to Claim 1, if appropriate with customary auxiliaries and additives, is converted into a suitable administration form.

6. Medicaments, comprising at least one compound of the general formula (I) according to Claim 1 in combination with organic nitrates or NO donors.

7. Medicaments, comprising at least one compound of the general formula (I) according to Claim 1 in combination with compounds which inhibit the degradation of cyclic guanosine monophosphate (cGMP).

8. Use of compounds of the general formula (I) according to Claim 1 for preparing medicaments.

9. Use of compounds of the general formula (I) according to Claim 1 for preparing medicaments for the treatment of cardiovascular diseases.

10. Use of compounds of the general formula (I) according to Claim 1 for preparing medicaments for the treatment of hypertension.

11. Use of compounds of the general formula (I) according to Claim 1 for preparing medicaments for the treatment of thromboembolic disorders and ischemia.

12. Use of compounds of the general formula (I) according to Claim 1 for preparing medicaments for the treatment of sexual dysfunction.

13. Use of compounds of the general formula (I) according to Claim 1 for preparing medicaments having antiinflammatory properties.

14. Use according to any of Claims 8 to 13 where the compounds of the general formula (I) according to Claim 1 are used in combination with organic nitrates or NO donors or in combination with compounds which inhibit the degradation of cyclic guanosine monophosphate (cGMP).

## Revendications

1. Dérivés substitués de pyrazole de formule générale (I) 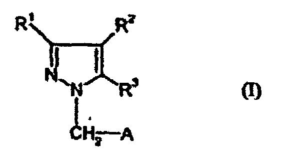 dans laquelle
R² et R³ forment avec la double liaison un noyau pyridyle ou pyrimidinyle,
A est un reste phényle ou un reste pyrimidyle, qui sont éventuellement substitués par du fluor, du chlore ou du brome,
R¹ est un reste de formule 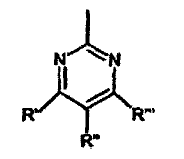
dans laquelle
R' est un groupe NH₂,
R" est un groupe morpholino, pipéridine, pipérazine, pyrrolidine, triazolyle ou thiomorpholino éventuellement substitué et
R"' représente l'hydrogène ou un groupe NH₂.

2. Composés suivant la revendication 1, dans lesquels R" est un groupe morpholinyle.

3. Procédé de production des composés de formule générale (I) suivant la revendication 1,
**caractérisé en ce que**
selon les différentes définitions des hétérocycles indiqués ci-dessus pour R² et R³.
[A] on transforme des composés de formule générale (II)
R¹-D (II)
dans laquelle
R¹ a la définition indiquée ci-dessus,
et
D représente des restes de formules 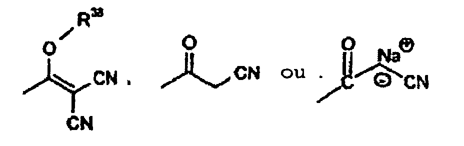
dans lesquelles
R³⁸ est un reste alkyle en C₁ à C₄,
par réaction avec des composés de formule générale (III)
A-CN₂-NH-NH₂ (III)
dans laquelle
A a la définition indiquée ci-dessus
dans des solvants inertes, éventuellement en présence d'une base, en composés de formule générale (IV) ou (IVa) 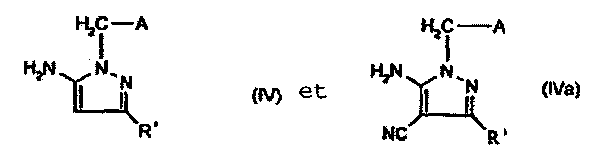 dans laquelle
A et R¹ ont la définition indiquée ci-dessus,
et dans le cas des composés de formule générale (IVa), on les cyclise ensuite avec des acides carboxyliques, des nitriles, des formamides ou des sels de guanidinium,
et dans le cas des composés de formule générale (IV), on les cyclise avec des dérivés 1,3-dicarbonylique, leurs sels, leurs tautomères, leurs énoléthers, ou leurs énamines, en présence d'acides et, le cas échéant, sous l'action de micro-ondes,
ou bien
[C] On fait réagir des composés de formule générale (VII) 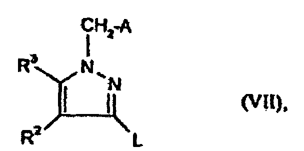 dans laquelle
A¹, R² et R³ ont la définition indiquée ci-dessus, et
L représente un reste de formule -SnR³⁹R⁴⁰R⁴¹, ZnR⁴², l'iode, le brome ou le groupe triflat,
où
R³⁹, R⁴⁰ et R⁴¹ sont identiques ou différents et désignent un reste alkyle linéaire ou ramifié ayant jusqu'à 4 atomes de carbone,
et
R⁴² représente un halogène,
avec des composés de formule générale (VIII)
R¹-T (VIII),
dans laquelle
R¹ a la définition indiquée ci-dessus
et
dans le cas de L = SnR³⁹R⁴⁰R⁴¹ ou ZnR⁴²
T est un groupe triflat, ou un halogène, avantageusement le brome,
et
dans le cas de L = iode, brome ou le groupe triflat
T r eprésente un reste de formule SnR^{39'}R^{40'}R^{41'} , ZnR^{42'} ou BR^{43'}R^{44'}
où
R^{39'} , R^{40'} , R^{41'} et R^{42'} ont la définition indiquée ci-dessus pour R³⁹, R⁴⁰ , R⁴¹ et R⁴² et y sont identiques ou en sont différents,
R^{43'} et R^{44'} sont identiques ou différents et représentent un groupe hydroxy, un reste aryloxy ayant 6 à 10 atomes de carbone ou un reste alkyle ou alkoxy linéaire ou ramifié ayant chacun jusqu'à 5 atomes de carbone, ou forment ensemble un noyau carbocyclique pentagonal ou hexagonal,
dans une réaction catalysée par le palladium dans des solvants inertes,
éventuellement en présence d'une base,
ou bien
[D] au cas où R¹ représente un reste pyrimidine éventuellement substitué,
on fait réagir des amidines de formule générale (IX), 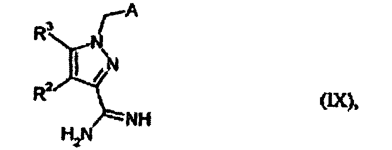 dans laquelle
A, R² et R³ ont la définition indiquée ci-dessus, par exemple avec des composés de formule générale (X), (Xa), (Xb) ou (Xc) 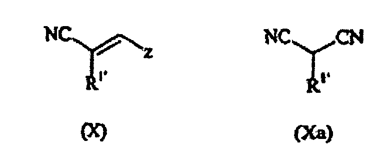 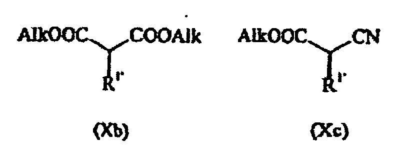 dans lesquelles
R^{1'} représente le reste cycloalkyle éventuellement substitué indiqué ci-dessus pour R¹,
Alk désigne un reste alkyle linéaire ou ramifié ayant jusqu'à 8 atomes de carbone, avantageusement jusqu'à quatre atomes de carbone ;
et
Z est un groupe NH₂, un groupe monoalkylamino ayant jusqu'à 7 atomes de carbone, un groupe dialkylamino ayant jusqu'à 7 atomes de carbone, un reste pipéridino ou morpholino lié par l'intermédiaire de l'azote, un groupe hydroxyle, un reste alkoxy ayant jusqu'à 7 atomes de carbone, acyloxy ayant jusqu'à 7 atomes de carbone ou aroyloxy ayant 6 à 10 atomes de carbone,
et on fait varier ou on introduit éventuellement les substituants indiqués pour X, Y, R¹, R², R³ et/ou A selon des modes opératoires usuels, avantageusement par acylation de groupes amino ou de groupes hydroxy libres, chloration, hydrogénation catalytique, réduction, oxydation, élimination de groupes protecteurs et/ou substitution nucléophile.

4. Médicament contenant au moins un composé de formule générale (I) suivant la revendication 1.

5. Procédé de préparation de médicaments, **caractérisé en ce qu'**on fait prendre une forme convenant pour l'administration à au moins un composé de formule (I) suivant la revendication 1, éventuellement avec des substances auxiliaires et des additifs usuels.

6. Médicament contenant au moins un composé de formule générale (I) suivant la revendication 1, en combinaison avec des nitrates organiques ou des donneurs de NO.

7. Médicament contenant au moins un composé de formule générale (I) suivant la revendication 1, en combinaison avec des composés qui inhibent la dégradation de guanosine monophosphate cyclique (GMPc).

8. Utilisation de composés de formule générale (I) suivant la revendication 1, pour la préparation de médicaments.

9. Utilisation de composés de formule générale (I) suivant la revendication 1, dans la préparation de médicaments destinés au traitement de maladies cardio-vasculaires.

10. Utilisation de composés de formule générale (I) suivant la revendication 1, dans la préparation de médicaments destinés au traitement de l'hypertonie.

11. Utilisation de composés de formule générale (I) suivant la revendication 1, dans la préparation de médicaments destinés au traitement de maladies thromboemboliques et d'ischémie.

12. Utilisation de composés de formule générale (I) suivant la revendication 1, dans la préparation de médicaments destinés au traitement d'un dysfonctionnement sexuel.

13. Utilisation de composés de formule générale (I) suivant la revendication 1, dans la préparation de médicaments doués de propriétés anti-inflammatoires.

14. Utilisation suivant l'une des revendications 8 à 13, dans laquelle les composés de formule générale (I) suivant la revendication 1 sont utilisés en combinaison avec des nitrates organiques ou des donneurs de NO ou en combinaison avec des composés qui inhibent la dégradation de guanosine monophosphate cyclique (GMPc).
